(19) **Europäisches Patentamt**
**European Patent Office**
**Office européen des brevets**

(11) **EP 4 644 561 A1**

(12) **EUROPEAN PATENT APPLICATION**

(43) Date of publication:
**05.11.2025 Bulletin 2025/45**

(51) International Patent Classification (IPC):
***C12Q 1/25*** *(2006.01)* ***G01N 33/542*** *(2006.01)*

(21) Application number: **24174120.6**

(52) Cooperative Patent Classification (CPC):
**G01N 33/542; C12Q 1/25**

(22) Date of filing: **03.05.2024**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Hochschule für Technik und Wirtschaft (HTW)**
**Berlin**
**10318 Berlin (DE)**

(72) Inventors:
• **FRANKE, Jacqueline**
**13187 Berlin (DE)**
• **RICHTER, Felix**
**10318 Berlin (DE)**

(74) Representative: **Geling, Andrea**
**ZSP Patentanwälte PartG mbB**
**Hansastraße 32**
**80686 München (DE)**

Remarks:
The complete document including Reference Table(s) and the Sequence Listing(s) can be downloaded from the EPO website

(54) **OPTICAL, CELL-BASED, HIGH-THROUGHPUT CAPABLE METHOD FOR THE DETERMINATION OF TELOMERE LENGTH AND DYNAMICS IN YEAST**

(57) The present invention relates to a method for determining telomere length. Further, the present invention relates to a method for identifying substances effecting telomere length. Furthermore, the present invention relates to a yeast cell which can be used in the above methods.

**Description**

The present invention relates to a method for determining telomere length. Further, the present invention relates to a method for identifying substances effecting telomere length. Furthermore, the present invention relates to a yeast cell which can be used in the above methods.

BACKGROUND OF THE INVENTION

Telomeres are the ends of chromosomes and protect them from degradation, end-to-end fusion, and recombination. Telomeres shorten in many cells (e.g. in all dividing human cells except germline and stem cells) with each cell division until a critical length is reached and senescence occurs. Telomeres are one of the nine hallmarks of aging. Telomeres are also closely linked to tumor growth, as telomeres shorten drastically in the first phases of cancer growth, but then lengthen again due to the activation of the telomere-lengthening enzyme telomerase, thus, helping the tumor to achieve unlimited ability to divide. Telomeres are therefore promising targets for active substances that can be used against ageing, age-related diseases, and cancer.

The mechanisms at the telomeres in yeast such as *Saccharomyces cerevisiae* and human cells are similar. Crucial processes at the telomeres were discovered in yeast, including the enzyme telomerase, which also prevents the critical shortening of telomeres in cancer cells. In addition, yeast is capable for high-throughput methods and enables rapid and cost-efficient screening of many thousands of active substances.

So far, only very time-consuming and material-intensive methods exist for measuring the telomere length. The classic method of telomere length measurement is telomeric restriction fragment (TRF) analysis, which requires radioactive labelling of telomeres and only allows a comparison of telomere length. Newer technologies allow the quantification of telomere length via nanopore sequencing. However, TRF analysis and nanopore sequencing are not suitable for high-throughput methods due to the cost and effort involved.

In order to carry out a large number of measurements, e.g. in the context of screening for active substances against cell ageing and cancer, methods are required that allow telomere length determination in a simple, time- and cost-saving manner.

The present inventors have implemented a new high-throughput capable method for measuring telomere length in yeast. The present inventors have, therefore, genomically labelled the telomere-binding proteins Rap1 and Rif2, which naturally form a protein complex at the telomere, so that increased binding of these proteins within the telomeric protein complex results in increased bioluminescence resonance energy transfer (BRET). For this purpose, Rif2 is fused with *Renilla reniformis*-luciferase (RLuc), which is known to emit light with maximum intensity at a wavelength of about 481 nm after conversion of the substrate coelentarazine and is used as an energy donor. Rap1 fused with a yellow fluorescent protein (YFP) acts, thereby, as an energy acceptor and emits light with a maximum intensity at a wavelength of about 527 nm when interacting with Rif1-RLuc. By measuring the BRET ratio (ratio of intensity of the emitted light at 505-590 nm to 430-485 nm), the determination of telomere length is possible. This determination is independent of the cell number or cell mass used. A quantification of the BRET signal, i.e. a translation into the actual number of nucleotides, is further possible through the calibration with measurements from long-read sequencing such as nanopore sequencing. The provided method is simple, time- and cost-effective and high-throughput capable and can, therefore, be used in high-throughput approaches.

SUMMARY OF THE INVENTION

In a first aspect, the present invention relates to a method for determining telomere length comprising the steps of:

(i) providing yeast cells expressing

(ia) a first telomere binding fusion protein comprising a bioluminescence donor, and
(ib) a second telomere binding fusion protein comprising a fluorescence acceptor,

(ii) incubating the yeast cells provided in (i) with a bioluminescence donor substrate,
(iii) detecting bioluminescence of the bioluminescence donor at a wavelength characteristic for the donor and detecting fluorescence of the fluorescence acceptor at a wavelength characteristic for the acceptor (in case of telomeric binding of the first telomere binding fusion protein and the second telomere binding fusion protein in physical proximity), and
(iv) determining the acceptor/donor ratio at both wavelengths, wherein the ratio at said wavelengths is indicative for the telomere length.

In a second aspect, the present invention relates to a method for identifying a substance effecting/having an effect on/influencing telomere length comprising the steps of:

(i) providing yeast cells expressing

(ia) a first telomere binding fusion protein comprising a bioluminescence donor, and
(ib) a second telomere binding fusion protein comprising a fluorescence acceptor,

(ii) incubating the yeast cells provided in (i) with a bioluminescence donor substrate and a substance potentially effecting /having an effect on/influencing telomere length,
(iii) detecting bioluminescence of the bioluminescence donor at a wavelength characteristic for the donor and detecting fluorescence of the fluorescence acceptor at a wavelength characteristic for the acceptor (in case of telomeric binding of the first telomere binding fusion protein and the second telomere binding fusion protein in physical proximity),
(iv) determining the acceptor/donor ratio at both wavelengths, and
(v) comparing the acceptor/donor ratio determined in (iv) with an acceptor/donor reference ratio (obtained under the same conditions but without the substance potentially effecting/having an effect on/influencing telomere length), wherein a difference between the ratio and the reference ratio indicates that the substance effects/has an effect on/influences telomere length.

In a third aspect, the present invention relates to a yeast cell expressing a first telomere binding fusion protein comprising a bioluminescence donor and a second telomere binding fusion protein comprising a fluorescence acceptor.

In a fourth aspect, the present invention relates to the use of the yeast cell of the third aspect for determining telomere length.

In a fifth aspect, the present invention relates to the use of the yeast cell of the third aspect for identifying a substance effecting/having an effect on/influencing telomere length.

This summary of the invention does not describe all features of the invention.

DETAILED DESCRIPTION OF THE INVENTION

<u>Definitions</u>

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety. Nothing herein is to be construed as an admission that the invention is not entitled to antedate such disclosure by virtue of prior invention.

In the following, the elements of the present invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. As used in this specification and the appended claims, the singular forms "a", "an", and "the" include plural referents, unless the content clearly dictates otherwise.

The term "consisting essentially of', as used herein, limits the scope of a claim to the specified materials or steps and those that do not materially affect the basic and novel characteristic(s) of the claimed invention. In other words, the term "consisting essentially of', as used herein, is generally construed to mean that the composition or formulation (a) necessarily includes the listed ingredients and (b) is open to unlisted ingredients that do not materially affect the basic and novel properties of the composition. Similarly, when "consisting essentially of' is used herein in a process claim, the claim requires that the listed steps are performed, but also may include unlisted steps that do not affect the basic and material properties of the process.

The term "about" when used in connection with a numerical value is meant to encompass numerical values within a range having a lower limit that is 5% smaller than the indicated numerical value and having an upper limit that is 5% larger than the indicated numerical value.

The terms "polypeptide" and "protein" are used interchangeably in the context of the present invention and refer to a long peptide-linked chain of amino acids.

Assays for telomere length are performed to provide useful information on the relative age and remaining proliferative capability of a wide variety of cell types in numerous tissues.

The term "telomere", as used herein, refers to a region of repetitive nucleotide sequences (telomeric repeats) associated with specialized proteins at the ends of linear chromosomes. Telomeres can also be designated as the tips/caps of chromosomes. They do not contain any gene. Telomeres are a widespread genetic feature most commonly found in eukaryotes such as mammals like humans and yeasts like *Saccharomyces cerevisiae.* In most, if not all species possessing them, they protect the terminal regions of chromosomal DNA from progressive degradation and ensure the integrity of linear chromosomes by preventing DNA repair systems from mistaking the very ends of the DNA strand for a double-strand break.

The telomeric repeat (5' to 3' toward the end) in *Saccharomyces cerevisiae* is TGTGGGTGTGGTG (from RNA template) or G(2-3)(TG)(1-6)T (consensus). In vertebrates like humans or mice, the telomeric repeat (5' to 3' toward the end) is TTAGGG. In both cases, the telomeric repeat is guanine (G)-rich.

Telomeres shorten in many cells (e.g. in all dividing human cells except germline and stem cells) with each cell division until a critical length is reached and senescence occurs. However, it is known that cancer cells, germ cells, and all of the studied eukaryotic microorganisms have the ability to correct this shortening with an enzyme called telomerase. The control of senescence by telomerase is still being investigated. Nevertheless, it has been shown that telomere loss may play a role in senescence, a scenario for which there is evidence in yeast such as *Saccharomyces cerevisiae.* The loss of telomeres might not be the only cause of what is called aging. The loss may also portend the onset of other pathological conditions, which may manifest themselves later in life-conditions such as Creutzfeldt-Jacob disease, Alzheimer's disease, wearing of joints, presbyopia, programmed cell death, cancer, etc., may contribute to aging.

The term "cellular senescence", as used herein, refers to a state of stable cell cycle arrest in which proliferating cells become resistant to growth-promoting stimuli, typically in response to DNA damage or in case telomeres have reached a critical length. Senescent cells are characterized by morphological and metabolic changes, chromatin reorganization, altered gene expression, and adoption of a pro-inflammatory phenotype known as the senescence-associated secretory phenotype (SASP). The biological role of senescence is complex as both protective and deleterious effects of senescent cells have been described, largely dependent upon physiological context. For example, while senescence has likely evolved as a mechanism to avoid malignant transformation of damaged cells, the onset of senescence may contribute to many age-associated pathologies, including cancer, tissue degeneration and inflammatory diseases.

The terms aging and cellular senescence cannot be used interchangeably. Aging is a progressive decline with time whereas senescence occurs throughout the lifespan, including during embryogenesis. The number of senescent cells increases with age, but senescence also plays an important role during development as well as during wound healing.

The term "telomere length (TL)", as used herein, represents a dynamic marker that reflects the genetic predisposition together with the environmental condition of an individual. The telomere length is closely related to longevity and a number of pathological conditions. An active role of telomere length in age-related human diseases arises because short telomeres increase the risk of diseases related to restricted cell proliferation and tissue degradation, such as cardio-vascular diseases (CVD). In contrast thereto, long telomeres increase the risk of diseases related to increased proliferative growth, such as major cancers. Therefore, the telomere length is considered as a potential biomarker for disease susceptibility or a possible target for a particular treatment.

Telomere length varies greatly between species, from approximately 300 base pairs in yeast to many kilobases in humans, and usually is composed of arrays of guanine (G)-rich, 6- to 14-base-pair-long repeats. Specifically, eukaryotic telomeres normally terminate with a 3'single-stranded-DNA overhang ranging from 24 to 500 nucleotides, which is essential for telomere maintenance and capping. Telomere length is maintained by a balance between processes that lengthen and those that shorten telomeres.

The term "end replication problem", as used herein, means the following: As mentioned above, the telomeres are shortened at each cell division. Reason for this is that during DNA replication, DNA polymerase cannot replicate the sequences present at the 3' ends of the parent strands. This is a consequence of its unidirectional mode of DNA synthesis:

it can only attach new nucleotides to an existing 3'-end (that is, synthesis progresses 5'-3') and, thus, it requires a primer to initiate replication. On the leading strand (oriented 5'-3' within the replication fork), DNA-polymerase continuously replicates from the point of initiation all the way to the strand's end with the primer (made of RNA) then being excised and substituted by DNA. The lagging strand, however, is oriented 3'-5' with respect to the replication fork so continuous replication by DNA-polymerase is impossible, which necessitates discontinuous replication involving the repeated synthesis of primers further 5' of the site of initiation. As the ends of linear DNA cannot be replicated completely during lagging strand DNA synthesis, telomeres are shortened at each cell division.

The "end replication problem" is exclusive to linear chromosomes as circular chromosomes do not have ends lying without reach of DNA-polymerases. While most prokaryotes rely on circular chromosomes, accordingly do not possess telomeres, eukaryotes such as mammals like humans and yeasts like *Saccharomyces cerevisiae* rely on linear chromosomes.

Apart from the end replication problem, different studies have shown that oxidative stress, environmental toxins, as well as psychological stress leads to telomere shortening.

As mentioned above, eukaryotic microorganisms such as yeasts like *Saccharomyces cerevisiae* have the ability to correct the telomer shortening with an enzyme called telomerase. Human cells are also capable of maintaining the length of telomeres with the telomerase enzyme. However, this capability is extinguished during embryonic differentiation in most somatic cells, but remains active in germline cells, activated lymphocytes and certain types of stem cell populations. During normal human growth and development, telomerase activity is precisely regulated by a number of genes in order to meet the proliferative demand of the specific cellular function. Cancer cells, on the other hand, can acquire the possibility of an infinite number of divisions by enhancing the telomerase activity, thus, restraining the shortening of telomeres, or with the mechanism of the alternative lengthening of telomeres (ALT).

The term "telomerase (also designated as telomeric reverse transcriptase (TERT))", as used herein, refers to a ribonucleoprotein enzyme complex that specifically elongates telomeres. More precisely, the telomerase consists of a complex of RNA and proteins. It brings along a repetitive RNA section (telomere repeat), which serves as a template for the reverse transcriptase when it lengthens the telomeres. A DNA polymerase then completes the complementary strand. In other words, the telomerase functions to elongate the guanine (G)-rich strand of eukaryotic, and preferably yeast, chromosomal termini by adding telomeric repeats to the 3'end of telomeres.

In the yeast *Saccharomyces cerevisiae,* the telomerase core enzyme minimally consists of the catalytic subunit Est2 and the RNA subunit TLC1. TLC1 provides a template for reverse transcription by Est2 and also functions as a scaffold for the accessory subunits, Est1, the Ku heterodimer, the $Sm_7$ heteroheptamer, and the Pop1/PopG/Pop7 components of RNase P/MRP. Although Est2 and TLC1 are sufficient to reconstitute enzymatic activity *in vitro,* the additional accessory proteins Est1 and Est3 are required for telomerase function *in vivo.* Another key player at yeast telomeres is the telomeric single-stranded (ss) DNA-binding protein Cdc13, which acts as a platform for proteins that cap the ends and coordinate the replication of both the telomeric G- and C-rich strands.

As mentioned above, telomeres are nucleoprotein complexes at the end of linear eukaryotic chromosomes which maintain the genome integrity by regulating telomere length, preventing recombination and end to end fusion events. Multiple proteins associate with telomeres and function in concert to carry out these functions. These proteins are called telomere binding proteins.

The term "telomere-binding protein", as used herein, refers to a protein which binds telomeric DNA in various species. The telomere-binding protein regulates the structure and function of telomeres through the formation of different complexes with telomeric DNA. The proteins Rap1, Rif1, and Rif2 belong, for example, to the telomere-binding proteins.

The term "Rap1-interacting factor 1 (Rifl)", as used herein, refers to a protein involved in telomere length regulation in yeast. Rif1 is conserved up to mammals. Rif1 was originally identified in yeast *Saccharomyces cerevisiae* as an interactor of the major telomere repeat sequence binding protein Rap1. Rif1 is a negative regulator of telomerase and together with another Rap1 interacting protein, Rif2, it controls the access of telomerase to telomere ends for replication and elongation of telomere sequences. Rif1 binds double-stranded regions with telomeric DNA-repeat sequences indirectly via association of Rif1 with Rap1.

The term "Rap1-interacting factor 2 (Rif2)", as used herein, refers to a protein involved in telomere length regulation in yeast. Rif2 is conserved up to mammals. Rif2 was originally identified in yeast *Saccharomyces cerevisiae,* as an interactor of the major telomere repeat sequence binding protein Rap1. Rif2 is a negative regulator of telomerase and together with another Rap1 interacting protein, Rif1, it controls the access of telomerase to telomere ends for replication and elongation of telomere sequences. Rif2 binds double-stranded regions with telomeric DNA-repeat sequences indirectly via association of Rif2 with Rap1.

The term "Rap1", as used herein, refers to the main protein that binds double-stranded telomeric DNA in *Saccharomyces cerevisiae.* The interaction with the telomeric DNA-repeat sequences occurs direct. Rap1 is essential for the maintenance of the telomeres of *Saccharomyces cerevisiae.* It binds *in vitro* to multiple sites found within the TG1-3 telomeric repeats. In addition to its known binding activity for double-stranded DNA, RAP1 binds sequence-specifically to the GT-strands. This indicates that RAP1 is the protein that binds to the telomeric terminal GT-tails.

Together with the telomere binding proteins Rif1 and Rif1, Rap1 forms a protective proteinaceous cap that regulates telomere length. For example, if a telomere is long, it binds many Rap1, Rif1, and Rif2 molecules. Thereby, the telomerase is inhibited and unwanted telomer lengthening is prevented. If the telomere becomes shorter, the number of Rap1, Rif1, and Rif2 molecule bound to telomeric sequences is decreasing. This, in turn, recruits the telomerase complex. The telomerase complex, which extends the telomeres, is active until enough Rap1 and Rif proteins can bind to the now long telomeric sequences again.

The term "bioluminescence", as used herein, refers to the ability of living organisms to produce visible light themselves or with the help of symbionts. Bioluminescence occurs widely in marine vertebrates and invertebrates as well as in some fungi, microorganisms including some bioluminescent bacteria, and terrestrial arthropods such as fireflies. In a general sense, the principal chemical reaction in bioluminescence involves a light-emitting molecule and an enzyme, e.g. a luciferase.

The term "bioluminescence donor", as used herein, refers to any molecule such as protein capable of acting on a suitable substrate to generate bioluminescence. There are a number of different bioluminescence donors that can be employed in this invention. Specifically, the bioluminescence donor is a bioluminescence donor protein (BDP). The bioluminescence donor may be a luciferase, aequorin, or obelin. The bioluminescence donor preferably used herein is a luciferase. More preferably, the luciferase is a *Renilla reniformis* (sea pansy) -luciferase (RLuc).

The term "fluorescence", as used herein, refers to the spontaneous emission of light shortly after a material is excited by light. The emitted photons are generally lower in energy than those previously absorbed. Physical systems in which fluorescence occurs are called fluorophores.

The term "fluorescence acceptor", as used herein, refers to any molecule which can accept energy emitted as a result of the activity of a bioluminescent donor protein, and re-emit it as light energy. There are a number of different fluorescent acceptors that can be employed in this invention. Specifically, the fluorescent acceptor is a fluorescent acceptor molecule (FAM). The FAM may be proteinaceous or non-proteinaceous.

The fluorescent acceptor may be a yellow fluorescent protein (YFP) or a green fluorescent protein 2 (GFP$^2$). The fluorescent acceptor preferably used herein is a yellow fluorescent protein (YFP). More preferably, the fluorescence acceptor is a yellow fluorescent protein (YFP) that has been mutated to have a higher affinity for luciferases (interacting YFP) as described in WO 2024/089297 (PCT/EP2023/080279). Specifically, the transient interaction of the mutated YFP and its higher quantum yield results in a stronger BRET signal at the same level of interaction between the proteins fused to donor and acceptor. This allows also the detection of weaker interactions in a broader dynamic signal range. Alternatively, an enhanced yellow fluorescent protein (EYFP) can be used. EYFP is a basic (constitutively fluorescent) green/yellow fluorescent protein published in 1996, derived from *Aequorea victoria.* It is reported to be a very rapidly-maturing weak dimer with high acid sensitivity.

The term "bioluminescence donor substrate", as used herein, refers to any molecule that is employed by the bioluminescent donor protein to generate luminescence. A preferred substrate is coelenterazine. Coelenterazine is a natural substance from the group of luciferins and serves as a substrate for the enzymes luciferase, aequorin, and obelin for the production of light (bioluminescence). Coelenterazine is converted in the presence of oxygen ($O_2$) into coelenteramide and carbon dioxide ($CO_2$). Alternatively, coelenterazine 400a (DeepBlue C) can be used. Coelenterazine 400a differs from coelenterazine in that it emits light of a shorter wavelength than native coelenterazine. This helps separate its emission peak from that of the Green Fluorescent Protein (GFP) to use RLuc as donor and GFP acceptor. This combination is called BRET2.

The term "bioluminescence resonance energy transfer (BRET)", as used herein, is based on the fact that the energy generated from a bioluminescence donor, such as a luciferase enzyme, can be used to excite a fluorescence acceptor when it is in close proximity to the bioluminescence donor, such as the luciferase enzyme. The technology involves the fusion of the bioluminescence donor (e.g. luciferase) and the fluorescence acceptor (e.g. yellow fluorescent protein (YFP) or green fluorescent protein 2 (GFP$^2$)) to/with proteins of interest. The co-expression of fusion constructs in living cells makes it possible to study their interaction quantitatively in real time. Energy transfer occurs by non-radiative dipole-dipole coupling from the donor to the acceptor in close proximity (typically within 10 nm), resulting in fluorescence emission at a characteristic wavelength. The energy emitted by the acceptor relative to the energy emitted by the donor is called the BRET signal or BRET ratio. It depends on the spectral properties, the ratio, distance and relative orientation of the donor and acceptor molecules as well as the strength and stability of the interaction between the proteins of interest.

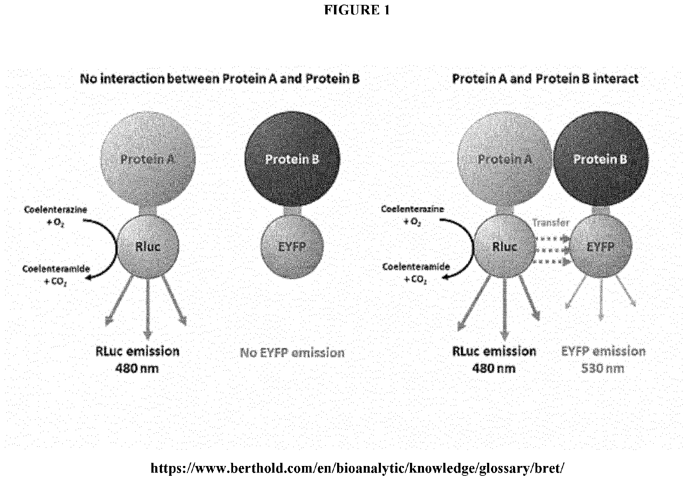

**FIGURE 1** is a schematic representation of a BRET assay.

BRET is similar to fluorescence resonance energy transfer (FRET), but in FRET the donor is also a fluorescence molecule that needs to be excited. Since BRET does not require an external light source to excite the donor, it has a very low background and does not suffer from problems often associated with FRET, such as autofluorescence, light scattering or photobleaching. Other advantages of BRET are that it is a non-radioactive and homogeneous technology and that the ratiometric signal minimizes interference from test conditions.

In the methods of the present invention, a yeast cell expressing a first telomere binding fusion protein comprising a bioluminescence donor and a second telomere binding fusion protein comprising a fluorescence acceptor is provided.

In these methods, bioluminescence of the bioluminescence donator and fluorescence of the fluorescence acceptor is only detected in case of telomeric binding of the first telomere binding fusion protein comprising a bioluminescence donator and the second telomere binding fusion protein comprising a fluorescence acceptor in physical proximity. In this respect, physical proximity means a distance of between 2 and 10 nm, e.g. 2, 3, 4, 5, 6, 7, 8, 9, or 10 nm, preferably as low as possible. The working distance of a donator/acceptor pair is determined by its Förster distance R0 which is the distance between donator and acceptor in which signal transduction works with 50 % efficiency. For the pair of RLuc and normal YFP the Förster distance has been measured to be 4.44 nm, which makes its usable working range of preferably 2.22 - 6.66 nm.

Preferably,

the first telomere binding fusion protein comprises Rap1-interacting factor 1 (Rifl) or Rap1-interacting factor 2 (Rif2) and a bioluminescence donator (e.g. luciferase), and the second telomere binding fusion protein comprises Rap1 and a fluorescence acceptor (e.g. yellow fluorescent protein (YFP) or green fluorescent protein 2 (GFP$^2$)), or

the first telomere binding fusion protein comprises Rap1 and a bioluminescence donator (e.g. luciferase) and the second telomere binding fusion protein comprises Rif1 or Rif2a and a fluorescence acceptor (e.g. yellow fluorescent protein (YFP) or green fluorescent protein 2 (GFP$^2$)).

More preferably, the first telomere binding fusion protein comprises Rif2 and a bioluminescence donator (e.g. luciferase) and the second telomere binding fusion protein comprises Rap1 and a fluorescence acceptor (e.g. yellow fluorescent protein (YFP) or green fluorescent protein 2 (GFP$^2$)).

As mentioned above, the bioluminescence of the bioluminescence donator at a wavelength characteristic for the donator and the fluorescence of the fluorescence acceptor at a wavelength characteristic for the acceptor (in case of telomeric binding of the first telomere binding fusion protein and the second telomere binding fusion protein in physical proximity) is detected in the methods of the present invention. Subsequently, the fluorescence/bioluminescence ratio or the acceptor/donator ratio at both wavelengths is determined. Said ratio at said wavelengths is indicative for the telomere length.

In this respect, the terms "fluorescence/bioluminescence ratio" and "acceptor/donator ratio" mean that the fluorescence signal intensity at a wavelength characteristic for the acceptor is divided by the bioluminescence signal intensity at a wavelength characteristic for the donator. Accordingly, in case of long telomeres, relatively more telomere binding fusion proteins, e.g. proteins encompassing Rif1, Rif2, and/or Rap1, can form protein complexes on the telomeric region so that fluorescence signal intensity is higher and the bioluminescence signal is lower, increasing the acceptor/donator signal ratio, while in case of shorter telomeres, relatively less telomere binding fusion proteins, e.g. proteins encompassing Rif1, Rif2, and/or Rap1 can bind each other at the telomeric region so that a higher ratio of the donator is not within close proximity to the acceptor, increasing the bioluminescence and decreasing the fluorescence signal intensity, leading to a lower acceptor/donator ratio. On the basis of the bioluminescence signal intensity, the fluorescence signal intensity and from the calculated ratio, relative telomere lengths can be determined. The absolute telomere length can also be calculated by correlating the ratio to a telomere length using a calibration curve. Preferably, the calibration curve is produced by long-read (nanopore) sequencing of telomeres of cells harvested directly after measuring their fluorescence/bioluminescence ratio or acceptor/donator ratio at the analysed wavelengths. Acceptor/donator ratios and absolute telomere length data from cells with different telomere length can be compared. From this a mathematical interdependency between those values can be inferred by regression. This interdependency can be used to infer telomere length from BRET ratio for unknown samples at the same experimental conditions.

The term "relative telomere length (TL)", as used herein, refers to a direct comparison of telomer length between cells in different conditions (especially treated/untreated) without knowing the absolute telomere length of any of them. For example, treatment of cells with substance A increased/decreased their telomere length compared to untreated cells.

The term "absolute telomere length (TL)", as used herein, refers to the quantification of telomere length of an organism in absolute units like nucleotides.

In one preferred embodiment of the present invention, Rif2 is fused with *Renilla reniformis*-luciferase (RLuc), which is known to emit light with maximum intensity at a wavelength of about 481 nm after conversion of the substrate coelentarazine and is used as an energy donor. Rap1 fused with a yellow fluorescent protein (YFP) acts, thereby, as an energy acceptor and emits light with a maximum intensity at a wavelength of about 527 nm when interacting with Rap1-RLuc. By measuring the BRET ratio (ratio of intensity of the emitted light at 505-590 nm to 430-485 nm), the determination of telomere length is possible.

The term "nanopore sequencing", as used herein, is a third generation approach used in the sequencing of biopolymers, specifically, polynucleotides in the form of DNA or RNA. Using nanopore sequencing, a single molecule of DNA or RNA can be sequenced without the need for Polymerase Chain Reaction (PCR) amplification or chemical labelling of the sample. Nanopore sequencing has the potential to offer relatively low-cost genotyping, high mobility for testing, and rapid processing of samples. Nanopore sequencing is the only sequencing technology to enable real-time analysis in fully scalable formats.

The principle of nanopore sequencing is as follows: The biological or solid-state membrane, where the nanopore is found, is surrounded by an electrolyte solution. The membrane splits the solution into two chambers. A bias voltage is applied across the membrane inducing an electric field that drives charged particles, in this case the ions, into motion. This effect is known as electrophoresis. For high enough concentrations, the electrolyte solution is well distributed and all the voltage drop concentrates near and inside the nanopore. This means charged particles in the solution only feel a force from the electric field when they are near the pore region. This region is often referred as the capture region. Inside the capture region, ions have a directed motion that can be recorded as a steady ionic current by placing electrodes near the membrane. Imagine now a nano-sized polymer such as DNA or protein placed in one of the chambers. This molecule also has a net charge that feels a force from the electric field when it is found in the capture region. The molecule approaches this capture region aided by Brownian motion and any attraction it might have to the surface of the membrane. Once inside the nanopore, the molecule translocates through via a combination of electro-phoretic, electro-osmotic and sometimes thermo-phoretic forces. Inside the pore the molecule occupies a volume that partially restricts the flow of ions, observed as an ionic current drop. Based on various factors such as geometry, size and chemical composition, the change in magnitude of the ionic current and the duration of the translocation will vary. The magnitude of the electric current density across a nanopore surface depends on the nanopores dimensions and the composition of DNA or RNA that is occupying the nanopore.

Sequencing was made possible because, passing through the channel of the nanopore, the samples cause characteristic changes in the density of the electric current flowing through the nanopore. The total charge flowing through a nanopore channel is equal to the surface integral of electric current density flux across the nanopore unit normal surfaces between times $t_i$ and $t_2$.

In other words, different molecules can be sensed and potentially identified based on the modulation in ionic current. A strand of DNA or RNA is made up of a sequence of different combinations of four nucleotide bases: A, T (or U for RNA), G and C. Each base that passes through the nanopore can be identified through the characteristic disruption it causes to the current in real-time. Not only the DNA or RNA sequence can be detected, but also DNA or RNA modifications can be quantified in this way.

The most important difference of this technique compared to other sequencing methods, is that the read length (the length of a DNA sequence that can be read on one molecule) is technically not limited. This allows sequencing and length quantification of long repetitive nucleotide sequences like telomeres.

The term "Renilla reniformis-luciferase (RLuc)", as used herein, refers to a protein from the sea pansy which emits light (at a wavelength of about 481 nm) in the presence of coelenterazine and oxygen ($O_2$). Specifically, it converts the substrate coelenterazine in the presence of oxygen ($O_2$) into coelenteramide and carbon dioxide ($CO_2$). Alternatively, the substrate coelenterazine 400a (DeepBlue C) can be used. The Renilla reniformis-luciferase (RLuc) has preferably an amino acid sequence according to SEQ ID NO: 1.

The term "green fluorescent protein (GFP)", as used herein, refers to a protein that exhibits green fluorescence when exposed to light in the blue to ultraviolet range. The label GFP traditionally refers to the protein first isolated from the jellyfish Aequorea victoria. However, diverse GFPs have been found in other organisms including corals, sea anemones, zoanithids, copepods, and lacelets. Preferably, green fluorescent protein 2 ($GFP^2$) is used herein. Green fluorescent protein 2 ($GFP^2$) differs from GFP in that the codons have been optimized for mammalian cells and that it has a F64L mutation that leads to a quicker folding compared to other enhanced GFP variants.

The term "yellow fluorescent protein (YFP)", as used herein, refers to a genetic mutant of the green fluorescent protein (GFP) originally derived from the jellyfish Aequorea victoria. Its excitation peak is at a wavelength of about 513 nm and its emission peak is at a wavelength of about 527 nm. Preferably, a modified YFP is used herein. The modified YFP differs from YFP in that it transiently interacts with luciferases and is generally brighter than other YFP variants. The transient interaction of the mutated YFP and its higher quantum yield results in a stronger BRET signal at the same level of interaction between the proteins fused to donator and acceptor. This allows the detection of weaker interactions in a broader dynamic signal range. The modified YFP has preferably an amino acid sequence according to SEQ ID NO: 2. Alternatively, enhanced yellow fluorescent protein (EYFP) can be used.

The term "substance effecting/having an effect on telomere length", as used herein, refers to any substance which is capable of influencing telomere length. For example, the substance can increase telomere length by at least 5%, at least 10%, at least 20%, at least 30%, or at least 40%, or the substance can decrease telomere length by at least 5%, at least 10%, at least 20%, at least 30%, or at least 40%. The substance may bind to DNA, specifically telomeric DNA, to telomere-binding proteins, and/or to telomerase RNA or protein components. The telomer-binding proteins include, but are not limited to, Rap1, Rif1, and Rif2. The substance effecting/having an effect on telomere length includes, but is not limited to, a nucleic acid molecule, protein, polypeptide, peptide, an antibody, small molecule, cofactor and the like. Preferably, the substance effecting/having an effect on telomere length is selected from the group consisting of a drug, foodstuff, food supplement, secondary metabolite of an organism, and another chemical compound. More preferably, the drug is an anti-cancer drug, a growth factor, G-quadruplex stabiliser/destabiliser, senolytic, senostatic, senomorphic or anti-aging drug/supplement.

The terms "fusion protein" or "hybrid protein" (literally, made of parts from different sources) are used interchangeably in the context of the present invention. They refer to a protein which is created through the joining of two or more genes that originally coded for separate proteins/peptides. Translation of this fusion gene results in a single chain polypeptide with functional properties derived from each of the original proteins/peptides.

The terms "recombinant fusion protein" or "recombinant hybrid protein" are used interchangeably in the context of the present invention. They refer to a single chain polypeptide which is created artificially by recombinant DNA technology.

In the methods of the present invention, a yeast cell is provided comprising/carrying a first telomere binding fusion protein and a second telomere binding fusion protein.

In one preferred embodiment,

the first telomere binding fusion protein comprises Rap1-interacting factor 1 (Rifl) or Rap1-interacting factor 2 (Rif2) and a bioluminescence donator and the second telomere binding fusion protein comprises Rap1 and a fluorescence acceptor, or

the first telomere binding fusion protein comprises Rap1 and a bioluminescence donator and the second telomere binding fusion protein comprises Rif1 or Rif2a and a fluorescence acceptor. In one more preferred embodiment, the first telomere binding fusion protein comprises Rif2 and a bioluminescence donator and the second telomere binding fusion protein comprises Rap1 and a fluorescence acceptor.

The first telomere binding fusion protein comprising Rif2 and a *Renilla reniformis*-luciferase (RLuc) as bioluminescence donator has preferably an amino acid sequence according to SEQ ID NO: 3.

The second telomere binding fusion protein comprising Rap1 and modified YFP as fluorescence acceptor has preferably an amino acid sequence according to SEQ ID NO: 4.

The fusion proteins of the present invention are produced genetically/by recombinant means, e.g. by (i) replacing the nucleotide sequence encoding the natural-occurring telomere binding protein, e.g. Rap1, Rif1, and/or Rif2, by a nucleotide sequence encoding a fusion protein composed of the telomere binding protein, e.g. Rap1, Rif1, and/or Rif2, and a bioluminescence donator or fluorescence acceptor, (ii) cloning the nucleotide sequence encoding the bioluminescence donator or fluorescence acceptor in-frame with the nucleotide sequence encoding the natural-occurring telomere binding protein, e.g. Rap1, Rif1, and/or Rif2, (iii) introducing DNA double strand breaks via CRISPR/Cas9 in the genomic sequence encoding the natural-occurring telomere binding protein and supplying a linear DNA cassette with homology to this area on both sides and the sequence encoding the fluorescent/bioluminescent protein in the middle, thus, allowing the cell to repair the double strand break and destroy the CRISPR/Cas target site by integrating the protein coding sequence to the chosen position in/on the coding sequence of the telomeric protein, (iv) introducing a similar homology directed repair construct with a selectable marker instead of using CRISPR/Cas9, or (v) transforming the cell with a self-replicating plasmid expressing the fusion protein, which can be combined with genomically knocking out the natural-occurring telomere binding protein.

The term "yeasts", as used herein, refers to eukaryotic, single-celled microorganisms classified as members of the fungus kingdom. The term "yeast cells", as used herein, relates to living yeast cells. Preferably, the yeast cells are selected from the genera *Saccharomyces, Rhodotorula, Schizosaccharomyces, Kluyveromyces, Pichia, Hansenula, Rhodosporidium, Candida, Yarrowia, Lipomyces* and *Cryptococcus.* More preferably, the yeast cells are *Saccharomyces cerevisiae* cells.

Specifically, the yeast described herein is a recombinant/genomically modified yeast or the yeast cells described herein are recombinant/genomically modified yeast cells.

The term "expression", as used herein, includes any step involved in the production of the fusion proteins including, but not limited to, transcription, post-transcriptional modification, translation, post-translational modification, and secretion. Expression can be measured-for example, to detect increased expression-by techniques known in the art, such as measuring levels of mRNA and/or translated polypeptide.

The term "in-frame", in the context of the present invention, means a configuration in which one coding sequence of a polynucleotide is placed at an appropriate position relative to the coding sequence of another polynucleotide such that both sequences are expressed as fusion.

The yeast cells are cultivated/propagated in yeast cell specific culture medium. The term "yeast cell culture medium", as used herein, relates to a medium which contains all nutrients and salts needed for the specific yeast strain. For *Saccharomyces cerevisiae* the most common complete culture medium is YPD consisting of 1 % Yeast Extract, 2 % Peptone and 2 % Glucose (w/v) in water.

The term "inducible promoter", as used herein, refers to a strong regulatory promoter that helps in the effective expression of the desired genes. The regulatory promoters are activated only when they receive a specific stimulus. Upon activation, they bind to the RNA polymerase and transcriptional factors. Thus, the transcription process is initiated.

Embodiments of the invention

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous, unless clearly indicated to the contrary.

The present inventors have implemented a new high-throughput capable method for measuring telomere length in yeast. The present inventors have, therefore, genomically labelled the telomere-binding proteins Rap1 and Rif2, which naturally form a protein complex at the telomere, so that increased binding of these proteins within the telomeric protein complex results in increased bioluminescence resonance energy transfer (BRET). For this purpose, Rif2 is fused with *Renilla reniformis-luciferase* (RLuc), which is known to emit light with maximum intensity at a wavelength of about 481 nm after conversion of the substrate coelentarazine and is used as an energy donor. Rap1 fused with a yellow fluorescent protein (YFP) acts, thereby, as an energy acceptor and emits light with a maximum intensity at a wavelength of about 527 nm when interacting with Rif1-RLuc. By measuring the BRET ratio (ratio of intensity of the emitted light at 505-590 nm to 430-485 nm), the determination of telomere length is possible. This determination is independent of the cell number or cell mass used. A quantification of the BRET signal, i.e. a translation into the actual number of nucleotides, is further possible through the calibration with measurements from long-read sequencing such as nanopore sequencing. The provided method is simple, time- and cost-effective and high-throughput capable and can, therefore, be used in high-throughput approaches.

Thus, in a first aspect, the present invention relates to a method for determining telomere length comprising the steps of:

(i) providing yeast cells expressing

(ia) a first telomere binding fusion protein comprising a bioluminescence donor, and
(ib) a second telomere binding fusion protein comprising a fluorescence acceptor,

(ii) incubating the yeast cells provided in (i) with a bioluminescence donor substrate,
(iii) detecting bioluminescence of the bioluminescence donor at a wavelength characteristic for the donor and detecting fluorescence of the fluorescence acceptor at a wavelength characteristic for the acceptor (in case of telomeric binding of the first telomere binding fusion protein and the second telomere binding fusion protein in physical proximity), and
(iv) determining the acceptor/donor ratio at both wavelengths, wherein the ratio at said wavelengths is indicative for the telomere length.

The yeast cells provided in (i) are recombinant/genetically modified yeast cells. The yeast cells provided in (i) are preferably part of/comprised in a culture/growth medium which allows yeast cell proliferation/propagation. Specifically, the culture/growth medium has the following composition: Ultra-pure water containing 1 % Yeast Extract, 2 % Peptone and 2 % Glucose (w/v) (YPD Medium).

In one embodiment, the incubation step (ii) encompasses:

growing the yeast cells (on/in a culture/growth medium),
diluting the cells, specifically to an OD of between 0.1 to 1 at 600 nm, and
adding a bioluminescence substrate to the yeast cells, specifically in the dark for at least 5 minutes, preferably for at least 5 minutes to maximum 30 minutes, e.g. 5, 6, 7, 8, 9, 10, 11, 12, 13, 14, 15, 16, 17, 18, 19, 20, 21, 22, 23, 24, 25, 26, 27, 28, 29, or 30 minutes, while more preferably shaking and incubating the cells at 30 °C.

Preferably, the yeast cells have an optical density of between 0.1 and 1, e.g. 0.1, 0.2, 0.3, 0.4, 0.5, 0.6, 0.7, 0.8, 0.9, or 1, at 600 nm at the time of adding the substrate in step (ii).

By telomeric binding of the first telomere binding fusion protein and the second telomere binding fusion protein in physical proximity, a bioluminescence resonance energy transfer (BRET) takes place so that bioluminescence of the bioluminescence donor as well as fluorescence of the fluorescence acceptor is detectable/can be detected in step (iii). In this case, physical proximity preferably means a distance between the first telomer binding fusion protein and the second telomere binding fusion protein of between 2 and 10 nm, preferably of between 2 and 7 nm, e.g. 1, 2, 3, 4, 5, 6, 7, 8, 9, or 10 nm.

Step (iv) can alternatively be worded as determining the fluorescence/bioluminescence ratio at both wavelengths, wherein the ratio at said wavelengths is indicative for the telomere length, or as determining the fluorescence of the fluorescence acceptor/bioluminescence of the bioluminescence donor ratio at both wavelengths, wherein the ratio at said wavelengths is indicative for the telomere length.

In case of long telomeres, relatively more telomere binding fusion proteins can bind each other at the telomeric region/telomeres so that the bioluminescence signal is quenched and is, therefore, lower compared to the fluorescence signal intensity, while in case of shorter telomeres, relatively less telomere binding fusion proteins can bind each other at the telomeric region/telomeres so that the bioluminescence signal is not transferred to the fluorescence acceptor and is higher compared to the fluorescence signal. On the basis of the bioluminescence signal intensity, the fluorescence signal intensity and from the calculated fluorescence/bioluminescence ratio, (relative) telomere lengths can be determined.

Preferably, the (absolute) telomere length is calculated by correlating the ratio of step (iv) to a telomere length using a calibration curve. More preferably, the calibration curve is produced by long read (nanopore) sequencing of telomeres having a defined fluorescence/bioluminescence ratio or acceptor/donator ratio at the analysed wavelengths. This calibration curve is obtained by preparing yeast cultures with known different telomere lengths, for example mutated strains with known reduced/increased telomere length or cells that have been exposed to stressors as ethanol or caffeine, that increase/reduce telomere length. For these cultures acceptor/donator ratio and telomere length is measured. These values are plotted against each other and a mathematical relationship is calculated by regression. This relationship is used to calculate absolute telomere length from acceptor/donator ratio for unknown samples in similar experimental conditions.

The ratio determined in step (iv) may also be compared to a reference ratio to calculate the telomere length. Thereby, the reference ratio is the ratio typically obtained for a specific yeast strain. The higher the ratio (compared to the reference ratio), the more interactions between the telomere binding fusion proteins at the telomeric region/telomeres are possible as the telomeric binding sites/telomeres are longer. The lower the ratio (compared to the reference ratio), the less interactions between the telomere binding fusion proteins at the telomeric region/telomeres are possible as the telomeric binding sites/telomeres are shorter.

In any case, a ratio higher than a reference ratio, which is the ratio typically obtained for a specific yeast strain, is indicative for long telomeres and a ratio lower than a reference ratio, which is a ratio typically obtained for a specific yeast strain, is indicative for short telomeres.

Preferably,

the first telomere binding fusion protein comprises Rap1-interacting factor 1 (Rifl) or Rap1-interacting factor 2 (Rif2) and a bioluminescence donor, and the second telomere binding fusion protein comprises Rap1 and a fluorescence acceptor, or

the first telomere binding fusion protein comprises Rap1 and a bioluminescence donor, and the second telomere binding fusion protein comprises Rif1 or Rif2 and a fluorescence acceptor.

More preferably,

the first telomere binding fusion protein comprises Rif2 and a bioluminescence donor and the second telomere binding fusion protein comprises Rap1 and a fluorescence acceptor. Rap1 is the main protein of the telomer binding protein complex that binds double-stranded telomeric DNA in yeast.

Rif1 and Rif2 are proteins normally recruited to telomeres by Rap1.

Thus, a method for determining telomere length which comprises the steps of:

(i) providing yeast cells expressing

(ia) a first telomere binding fusion protein comprising Rif2 and a bioluminescence donor, and

(ib) a second telomere binding fusion protein comprising Rap1 and a fluorescence acceptor,

(ii) incubating the yeast cells provided in (i) with a bioluminescence donor substrate,

(iii) detecting bioluminescence of the bioluminescence donor at a wavelength characteristic for the donor and detecting fluorescence of the fluorescence acceptor at a wavelength characteristic for the acceptor (in case of telomeric binding of the first telomere binding fusion protein and the second telomere binding fusion protein in physical proximity), and

(iv) determining the acceptor/donor ratio at both wavelengths, wherein the ratio at said wavelengths is indicative for the telomere length

is more preferred.

Particularly, the first telomere binding fusion protein and the second telomere binding fusion protein bind to double-stranded regions comprising telomeric DNA-repeat sequences. More particularly, the (first/second) telomere binding fusion protein directly binds via Rap 1 double-stranded regions with telomeric DNA-repeat sequences and the (first/second) telomere binding fusion protein indirectly binds via Rif1 or Rif2 double-stranded regions with telomeric DNA-repeat sequences by association of Rif1 or Rif2 with Rap1.

In order to produce the first telomere binding fusion protein and/or the second telomere binding fusion protein, it is possible to integrate the (heterologous) nucleotide sequences encoding the bioluminescence donor and the fluorescence acceptor into the yeast cell genome. Therefore,

> the (heterologous) nucleotide sequence encoding the bioluminescence donor is preferably cloned in-frame with (the nucleotide sequence encoding) Rif1, Rif2, or Rap1, and/or
> the (heterologous) nucleotide sequence encoding the fluorescence acceptor is preferably cloned in-frame with (the nucleotide sequence encoding) Rif1, Rif2, or Rap1.

The (heterologous) nucleotide sequence encoding the bioluminescence donor may be cloned in-frame with (the nucleotide sequence encoding) Rif2 such that it is fused to the N-terminus and/or C-terminus of Rif2 in the final fusion protein. In addition, the (heterologous) nucleotide sequence encoding the fluorescence acceptor may be cloned in-frame with (the nucleotide sequence encoding) Rap1 such that it is fused to the N-terminus and/or C-terminus of Rap1 in the final fusion protein. More preferably, the (heterologous) nucleotide sequence encoding the bioluminescence donor is cloned in-frame with (the nucleotide sequence encoding) Rif2 such that it is fused to the N-terminus of Rif2 in the final fusion protein, and/or the (heterologous) nucleotide sequence encoding the fluorescence acceptor is cloned in-frame with (the nucleotide sequence encoding) Rap1 such that it is fused to the C-terminus of Rap1 in the final fusion protein.

It is also possible to produce the first telomere binding fusion protein and/or the second telomere binding fusion protein by replacing the nucleotide sequence encoding the natural-occurring telomere binding protein, e.g. Rap1, Rif1, and/or Rif2, by a nucleotide sequence encoding a fusion protein composed of the telomere binding protein, e.g. Rap1, Rif1, and/or Rif2, and a bioluminescence donor or fluorescence acceptor.

The bioluminescence donor may be localized/present at the N-terminus and/or C-terminus of Rif1 or Rif2. In addition, the fluorescence acceptor may be localized/present at the N-terminus and/or C-terminus of Rap1. Preferably, the bioluminescence donor is present at the N-terminus of Rif2, and/or the fluorescence acceptor is present at the C-terminus of Rap1.

It is preferred that the bioluminescence donor is a luciferase. It is more preferred that the luciferase is *Renilla reniformis*-luciferase (RLuc). The *Renilla reniformis*-luciferase (RLuc) has preferably an amino acid sequence according to SEQ ID NO: 1.

It is further preferred that the fluorescence acceptor is a yellow fluorescent protein (YFP) or green fluorescent protein 2 (GFP$^2$). It is more preferred that the yellow fluorescent protein (YFP) is an enhanced yellow fluorescent protein (EYFP) or a modified yellow fluorescent protein (YFP). The modified YFP has been mutated to have a higher affinity for luciferases (interacting YFP) as described in WO 2024/089297 (PCT/EP2023/080279). Specifically, the transient interaction of the mutated YFP and its higher quantum yield results in a stronger BRET signal at the same level of interaction between the proteins fused to donor and acceptor. This allows the detection of weaker interactions in a broader dynamic signal range. The modified YFP has preferably an amino acid sequence according to SEQ ID NO: 2.

It is also preferred that the bioluminescence donor substrate is coelenterazine or coelenterazine 400a (DeepBlue C).

The first telomere binding fusion protein comprising Rif2 and a *Renilla reniformis*-luciferase (RLuc) as bioluminescence donor has preferably an amino acid sequence according to SEQ ID NO: 3.

The second telomere binding fusion protein comprising Rap1 and modified YFP as fluorescence acceptor has preferably an amino acid sequence according to SEQ ID NO: 4.

The above method is performed with living yeast cells. Preferably, the yeast cells are selected from the genera *Saccharomyces, Rhodotorula, Schizosaccharomyces, Kluyveromyces, Pichia, Hansenula, Rhodosporidium, Candida, Yarrowia, Lipomyces* and *Cryptococcus*. More preferably, the yeast cells are *Saccharomyces cerevisiae* cells.

Thus, a method for determining telomere length which comprises the steps of:

> (i) providing *Saccharomyces cerevisiae* cells expressing
>
> > (ia) a first telomere binding fusion protein comprising Rif2 and a luciferase such as a *Renilla reniformis*-luciferase (RLuc) as bioluminescence donor, and
> > (ib) a second telomere binding fusion protein comprising Rap1 and a yellow fluorescent protein (YFP) such as EYFP or modified YFP as fluorescence acceptor,
>
> (ii) incubating the *Saccharomyces cerevisiae* cells provided in (i) with coelenterazine or coelenterazine 400a (DeepBlue C) as bioluminescence donor substrate,
> (iii) detecting bioluminescence of the bioluminescence donor at a wavelength characteristic for the donor and

detecting fluorescence of the fluorescence acceptor at a wavelength characteristic for the acceptor (in case of telomeric binding of the first telomere binding fusion protein and the second telomere binding fusion protein in physical proximity), and
(iv) determining the acceptor/donator ratio at both wavelengths, wherein the ratio at said wavelengths is indicative for the telomere length

is even more preferred.
In this method, the bioluminescence donator is preferably fused to/present at the N-terminus of Rif2, and/or the fluorescence acceptor is preferably fused to/present at the C-terminus of Rap1.

In one still even more preferred embodiment,

the bioluminescence of luciferase is detected at a wavelength of between 430 and 485 nm, preferably at a wavelength of 481 nm, and/or
the fluorescence of YFP is detected at a wavelength of between 505 and 590 nm, preferably at a wavelength of 527 nm.

Finally, the BRET ratio (the ratio of intensity of the emitted light) at 505-590 nm to 430-485 nm is determined.
Thus, a method for determining telomere length which comprises the steps of:

(i) providing *Saccharomyces cerevisiae* cells expressing

(ia) a first telomere binding fusion protein comprising Rif2 and a luciferase such as a *Renilla reniformis-luciferase* (RLuc) as bioluminescence donator, and
(ib) a second telomere binding fusion protein comprising Rap1 and a yellow fluorescent protein (YFP) such as EYFP or modified YFP as fluorescence acceptor,

(ii) incubating the *Saccharomyces cerevisiae* cells provided in (i) with coelenterazine or coelenterazine 400a (DeepBlue C) as bioluminescence donator substrate,
(iii) detecting bioluminescence of the bioluminescence donator at a wavelength around its emission peak, particularly between 430 and 485 nm for RLuc, and detecting fluorescence of the fluorescence acceptor at a wavelength around its emission peak, particularly between 505 and 590 nm for YFP (in case of telomeric binding of the first telomere binding fusion protein and the second telomere binding fusion protein in physical proximity), and
(iv) determining the acceptor/donator ratio at both wavelengths (for example 505-590 nm to 430-485 nm), wherein the ratio at said wavelengths is indicative for the telomere length

is still even more preferred.
In this method, the bioluminescence donator is preferably fused to/present at the N-terminus of Rif2, and/or the fluorescence acceptor is preferably fused to/present at the C-terminus of Rap1.
The yeast cells used in the above method may not express a functional telomerase or express telomerase components under an inducible promoter.
The method of the first aspect of the present invention can be carried out as high-throughput method allowing the analysis of two or more samples in parallel, e.g. of 96 samples in a 96-well-plate.
In the second aspect, the present invention relates to a method for identifying a substance effecting/having an effect on/influencing telomere length comprising the steps of:

(i) providing yeast cells expressing

(ia) a first telomere binding fusion protein comprising a bioluminescence donator, and
(ib) a second telomere binding fusion protein comprising a fluorescence acceptor,

(ii) incubating the yeast cells provided in (i) with a bioluminescence donator substrate and a substance potentially effecting /having an effect on/influencing telomere length,
(iii) detecting bioluminescence of the bioluminescence donator at a wavelength characteristic for the donator and detecting fluorescence of the fluorescence acceptor at a wavelength characteristic for the acceptor (in case of telomeric binding of the first telomere binding fusion protein and the second telomere binding fusion protein in physical proximity),
(iv) determining the acceptor/donator ratio at both wavelengths, and

(v) comparing the acceptor/donator ratio determined in (iv) with an acceptor/donator reference ratio (obtained under the same conditions but without the substance potentially effecting/having an effect on/influencing telomere length), wherein a difference between the ratio and the reference ratio indicates that the substance effects/has an effect on/influences telomere length.

The yeast cells provided in (i) are recombinant/genetically modified yeast cells. The yeast cells provided in (i) are preferably part of/comprised in a culture/growth medium which allows yeast cell proliferation/propagation. Specifically, the culture/growth medium has the following composition: Ultra-pure water containing 1 % Yeast Extract, 2 % Peptone and 2 % Glucose (w/v) (YPD Medium).

In one embodiment, the incubation step (ii) encompasses:

growing the yeast cells (on/in a culture/growth medium) in the presence of a substance potentially effecting/having an effect on/influencing telomere length (for a time period expected to be sufficient for physiological changes, typically a few days, e.g. between 1 and 10 days,
such as 1, 2, 3, 4, 5, 6, 7, 8, 9, or 10 day(s)),
diluting the cells, specifically to an OD of between 0.1 to 1 at 600 nm, and
adding a bioluminescence substrate to the yeast cells, specifically in the dark for at least 5 minutes, preferably for at least 5 minutes to maximum 30 minutes, e.g. 5, 6, 7, 8, 9, 10, 11, 12, 13, 14, 15, 16, 17, 18, 19, 20, 21, 22, 23, 24, 25, 26, 27, 28, 29, or 30 minutes, while more preferably shaking and incubating the cells at 30 °C.

Preferably, the yeast cells have an optical density of between 0.1 and 1, e.g. 0.1, 0.2, 0.3, 0.4, 0.5, 0.6, 0.7, 0.8, 0.9, or 1, at 600 nm at the time of adding the substrate in step (ii).

By telomeric binding of the first telomere binding fusion protein and the second telomere binding fusion protein in physical proximity, a bioluminescence resonance energy transfer (BRET) takes place so that bioluminescence of the bioluminescence donator as well as fluorescence of the fluorescence acceptor is detectable/can be detected in step (iii). In this case, physical proximity preferably means a distance between the first telomer binding fusion protein and the second telomere binding fusion protein of between 2 and 10 nm, preferably of between 2 and 7 nm, e.g. 1, 2, 3, 4, 5, 6, 7, 8, 9, or 10 nm.

Step (iv) can alternatively be worded as determining the fluorescence/bioluminescence ratio at both wavelengths, or as determining the fluorescence of the fluorescence acceptor/bioluminescence of the bioluminescence donator ratio at both wavelengths.

In case of long telomeres, relatively more telomere binding fusion proteins can bind each other at the telomeric region/telomeres so that more of the bioluminescence signal is transferred to the fluorescence acceptor and fluorescence signal intensity is higher compared to bioluminescence signal intensity, while in case of shorter telomeres, relatively less telomere binding fusion proteins can bind each other at the telomeric region/telomeres so that less of the bioluminescence signal is transferred to the fluorescent acceptor and fluorescence signal intensity is lower compared to the bioluminescence signal intensity. On the basis of the bioluminescence signal intensity and the fluorescence signal intensity, the acceptor/donator ratio is determined in step (iv).

In step (v), the acceptor/donator ratio determined in step (iv) is compared with an acceptor/donator reference ratio (obtained under the same conditions but without the substance potentially effecting/having an effect on/influencing telomere length), wherein a difference between the ratio and the reference ratio indicates that the substance effects/has an effect on/influences telomere length.

The term "under the same conditions" specifically means that the yeast cell used for the establishment of the reference ratio is cultivated/treated like the yeast cell used for the establishment of the ratio, e.g. the cultivation is carried out at the same temperature, with the same culture/growth medium, at the same humidity, for the same time, and to the same optical density.

Preferably,

a ratio (acceptor/donator) above the reference ratio (acceptor/donator) indicates that the substance increases telomere length/positively effects telomer length, or
a ratio (acceptor/donator) below the reference ratio (acceptor/donator) indicates that the substance decreases telomere length/negatively effects telomer length.

For example, the substance can increase telomere length by at least 5%, at least 10%, at least 20%, at least 30%, or at least 40%, or the substance can decrease telomere length by at least 5%, at least 10%, at least 20%, at least 30%, or at least 40%.

Thus, a method for identifying a substance effecting/having an effect on/influencing telomere length which comprises the steps of:

(i) providing yeast cells expressing

(ia) a first telomere binding fusion protein comprising a bioluminescence donator, and
(ib) a second telomere binding fusion protein comprising a fluorescence acceptor,

(ii) incubating the yeast cells provided in (i) with a bioluminescence donator substrate and a substance potentially effecting /having an effect on/influencing telomere length,
(iii) detecting bioluminescence of the bioluminescence donator at a wavelength characteristic for the donator and detecting fluorescence of the fluorescence acceptor at a wavelength characteristic for the acceptor (in case of telomeric binding of the first telomere binding fusion protein and the second telomere binding fusion protein in physical proximity),
(iv) determining the acceptor/donator ratio at both wavelengths, and
(v) comparing the acceptor/donator ratio determined in (iv) with an acceptor/donator reference ratio (obtained under the same conditions but without the substance potentially effecting/having an effect on/influencing telomere length), wherein

a ratio (acceptor/donator) above the reference ratio (acceptor/donator) indicates that the substance increases telomere length/positively effects telomer length, or
a ratio (acceptor/donator) below the reference ratio (acceptor/donator) indicates that the substance decreases telomere length/negatively effects telomer length

is preferred.

It is particularly preferred that

the first telomere binding fusion protein comprises Rap1-interacting factor 1 (Rifl) or Rap1-interacting factor 2 (Rif2) and a bioluminescence donator, and the second telomere binding fusion protein comprises Rap1 and a fluorescence acceptor, or
the first telomere binding fusion protein comprises Rap1 and a bioluminescence donator, and the second telomere binding fusion protein comprises Rif1 or Rif2 and a fluorescence acceptor.

It is particularly more preferred that
the first telomere binding fusion protein comprises Rif2 and a bioluminescence donator and the second telomere binding fusion protein comprises Rap1 and a fluorescence acceptor.
Rap1 is the main protein of the telomer binding protein complex that binds double-stranded telomeric DNA in yeast.
Rif1 and Rif2 are proteins normally recruited to telomeres by Rap1.

Thus, a method for identifying a substance effecting/having an effect on/influencing telomere length which comprises the steps of:

(i) providing yeast cells expressing

(ia) a first telomere binding fusion protein comprising Rif2 and a bioluminescence donator, and
(ib) a second telomere binding fusion protein comprising Rap1 and a fluorescence acceptor,

(ii) incubating the yeast cells provided in (i) with a bioluminescence donator substrate and a substance potentially effecting /having an effect on/influencing telomere length,
(iii) detecting bioluminescence of the bioluminescence donator at a wavelength characteristic for the donator and detecting fluorescence of the fluorescence acceptor at a wavelength characteristic for the acceptor (in case of telomeric binding of the first telomere binding fusion protein and the second telomere binding fusion protein in physical proximity),
(iv) determining the acceptor/donator ratio at both wavelengths, and
(v) comparing the acceptor/donator ratio determined in (iv) with an acceptor/donator reference ratio (obtained under the same conditions but without the substance potentially effecting/having an effect on/influencing telomere length), wherein

a ratio (acceptor/donator) above the reference ratio (acceptor/donator) preferably indicates that the substance increases telomere length/positively effects telomer length, or
a ratio (acceptor/donator) below the reference ratio (acceptor/donator) preferably indicates that the substance decreases telomere length/negatively effects telomer length

is more preferred.

For example, the substance can increase telomere length by at least 5%, at least 10%, at least 20%, at least 30%, or at least 40%, or the substance can decrease telomere length by at least 5%, at least 10%, at least 20%, at least 30%, or at least 40%.

Particularly, the first telomere binding fusion protein and the second telomere binding fusion protein bind to double-stranded regions comprising telomeric DNA-repeat sequences. More particularly, the (first/second) telomere binding fusion protein directly binds via Rap1 double-stranded regions with telomeric DNA-repeat sequences and the (first/second) telomere binding fusion protein indirectly binds via Rif1 or Rif2 double-stranded regions with telomeric DNA-repeat sequences by association of Rif1 or Rif2 with Rap1.

In order to produce the first telomere binding fusion protein and/or the second telomere binding fusion protein, it is possible to integrate the (heterologous) nucleotide sequences encoding the bioluminescence donor and the fluorescence acceptor into the yeast cell genome. Therefore,

the (heterologous) nucleotide sequence encoding the bioluminescence donor is preferably cloned in-frame with (the nucleotide sequence encoding) Rif1, Rif2, or Rap1, and/or
the (heterologous) nucleotide sequence encoding the fluorescence acceptor is preferably cloned in-frame with (the nucleotide sequence encoding) Rif1, Rif2, or Rap1.

The (heterologous) nucleotide sequence encoding the bioluminescence donor may be cloned in-frame with (the nucleotide sequence encoding) Rif2 such that it is fused to the N-terminus and/or C-terminus of Rif2 in the final fusion protein. In addition, the (heterologous) nucleotide sequence encoding the fluorescence acceptor may be cloned in-frame with (the nucleotide sequence encoding) Rap1 such that it is fused to the N-terminus and/or C-terminus of Rap1 in the final fusion protein. More preferably, the (heterologous) nucleotide sequence encoding the bioluminescence donor is cloned in-frame with (the nucleotide sequence encoding) Rif2 such that it is fused to the N-terminus of Rif2 in the final fusion protein, and/or the (heterologous) nucleotide sequence encoding the fluorescence acceptor is cloned in-frame with (the nucleotide sequence encoding) Rap1 such that it is fused to the C-terminus of Rap1 in the final fusion protein.

It is also possible to produce the first telomere binding fusion protein and/or the second telomere binding fusion protein by replacing the nucleotide sequence encoding the natural-occurring telomere binding protein, e.g. Rap1, Rif1, and/or Rif2, by a nucleotide sequence encoding a fusion protein composed of the telomere binding protein, e.g. Rap1, Rif1, and/or Rif2, and a bioluminescence donor or fluorescence acceptor.

The bioluminescence donor may be localized/present at the N-terminus and/or C-terminus of Rif1 or Rif2. In addition, the fluorescence acceptor may be localized/present at the N-terminus and/or C-terminus of Rap1. Preferably, the bioluminescence donor is present at the N-terminus of Rif2, and/or the fluorescence acceptor is present at the C-terminus of Rap 1.

It is preferred that the bioluminescence donor is a luciferase. It is more preferred that the luciferase is *Renilla reniformis-luciferase* (RLuc). The *Renilla reniformis-luciferase* (RLuc) has preferably an amino acid sequence according to SEQ ID NO: 1.

It is further preferred that the fluorescence acceptor is a yellow fluorescent protein (YFP) or green fluorescent protein 2 (GFP[2]). It is more preferred that the yellow fluorescent protein (YFP) is an enhanced yellow fluorescent protein (EYFP) or a modified yellow fluorescent protein (YFP). The modified YFP has been mutated to have a higher affinity for luciferases (interacting YFP) as described in WO 2024/089297 (PCT/EP2023/080279). Specifically, the transient interaction of the mutated YFP and its higher quantum yield results in a stronger BRET signal at the same level of interaction between the proteins fused to donor and acceptor. This allows the detection of weaker interactions in a broader dynamic signal range. The modified YFP has preferably an amino acid sequence according to SEQ ID NO: 2.

It is also preferred that the bioluminescence donor substrate is coelenterazine or coelenterazine 400a (DeepBlue C).

The first telomere binding fusion protein comprising Rif2 and a *Renilla reniformis*-luciferase (RLuc) as bioluminescence donor has preferably an amino acid sequence according to SEQ ID NO: 3.

The second telomere binding fusion protein comprising Rap1 and modified YFP as fluorescence acceptor has preferably an amino acid sequence according to SEQ ID NO: 4.

The above method is performed with living yeast cells. Preferably, the yeast cells are selected from the genera *Saccharomyces, Rhodotorula, Schizosaccharomyces, Kluyveromyces, Pichia, Hansenula, Rhodosporidium, Candida, Yarrowia, Lipomyces* and *Cryptococcus.* More preferably, the yeast cells are *Saccharomyces cerevisiae* cells.

Thus, a method for identifying a substance effecting/having an effect on/influencing telomere length which comprises the steps of:

(i) providing *Saccharomyces cerevisiae* cells expressing

(ia) a first telomere binding fusion protein comprising Rif2 and a luciferase such as a *Renilla reniformis-luciferase* (RLuc) as bioluminescence donator, and

(ib) a second telomere binding fusion protein comprising Rap1 and a yellow fluorescent protein (YFP) such as EYFP or modified YFP as fluorescence acceptor,

(ii) incubating the *Saccharomyces cerevisiae* cells provided in (i) with coelenterazine or coelenterazine 400a (DeepBlue C) as bioluminescence donator substrate and a substance potentially effecting /having an effect on/influencing telomere length,

(iii) detecting bioluminescence of the bioluminescence donator at a wavelength characteristic for the donator and detecting fluorescence of the fluorescence acceptor at a wavelength characteristic for the acceptor (in case of telomeric binding of the first telomere binding fusion protein and the second telomere binding fusion protein in physical proximity),

(iv) determining the acceptor/donator ratio at both wavelengths, and

(v) comparing the acceptor/donator ratio determined in (iv) with an acceptor/donator reference ratio (obtained under the same conditions but without the substance potentially effecting/having an effect on/influencing telomere length), wherein

a ratio (acceptor/donator) above the reference ratio (acceptor/donator) preferably indicates that the substance increases telomere length/positively effects telomer length, or

a ratio (acceptor/donator) below the reference ratio (acceptor/donator) preferably indicates that the substance decreases telomere length/negatively effects telomer length

is even more preferred.

In this method, the bioluminescence donator is preferably fused to/present at the N-terminus of Rif2, and/or the fluorescence acceptor is preferably fused to/present at the C-terminus of Rap1.

In one still even more preferred embodiment,

the bioluminescence of luciferase is detected at a wavelength of between 430 and 485 nm, preferably at a wavelength of 481 nm, and/or

the fluorescence of YFP is detected at a wavelength of between 505 and 590 nm, preferably at a wavelength of 527 nm.

Finally, the BRET ratio (the ratio of intensity of the emitted light) at 505-590 nm to 430-485 nm is determined.

Thus, a method for identifying a substance effecting/having an effect on/influencing telomere length which comprises the steps of:

(i) providing *Saccharomyces cerevisiae* cells expressing

(ia) a first telomere binding fusion protein comprising Rif2 and a luciferase such as a *Renilla reniformis-luciferase* (RLuc) as bioluminescence donator, and

(ib) a second telomere binding fusion protein comprising Rap1 and a yellow fluorescent protein (YFP) such as EYFP or modified YFP as fluorescence acceptor,

(ii) incubating the *Saccharomyces cerevisiae* cells provided in (i) with coelenterazine or coelenterazine 400a (DeepBlue C) as bioluminescence donator substrate and a substance potentially effecting /having an effect on/influencing telomere length,

(iii) detecting bioluminescence of the bioluminescence donator at a wavelength of between 430 and 485 nm and detecting fluorescence of the fluorescence acceptor at a wavelength of between 505 and 590 nm (in case of telomeric binding of the first telomere binding fusion protein and the second telomere binding fusion protein in physical proximity),

(iv) determining the acceptor/donator ratio at both wavelengths (at 505-590 nm to 430-485 nm), and

(v) comparing the acceptor/donator ratio determined in (iv) with an acceptor/donator reference ratio (obtained under the same conditions but without the substance potentially effecting/having an effect on/influencing telomere length), wherein

a ratio (acceptor/donator) above the reference ratio (acceptor/donator) preferably indicates that the substance increases telomere length/positively effects telomer length, or

a ratio (acceptor/donator) below the reference ratio (acceptor/donator) preferably indicates that the substance decreases telomere length/negatively effects telomer length

is still even more preferred.

In this method, the bioluminescence donor is preferably fused to/present at the N-terminus of Rif2, and/or the fluorescence acceptor is preferably fused to/present at the C-terminus of Rap1.

The substance potentially effecting /having an effect on/influencing telomere length may bind to DNA, specifically telomeric DNA, to telomere-binding proteins, and/or to telomerase RNA or DNA components. The telomer-binding proteins include, but are not limited to, Rap1, Rif1, and Rif2. The substance effecting/having an effect on telomere length includes, but is not limited to, a nucleic acid molecule, protein, polypeptide, peptide, antibody, small molecule, cofactor and the like. Preferably, the substance effecting/having an effect on telomere length is selected from the group consisting of a drug, foodstuff, food supplement, secondary metabolite of an organism, and another chemical compound. More preferably, the drug is an anti-cancer drug, a growth factor, G-quadruplex stabiliser/destabiliser, senolytic, senostatic, senomorphic, or anti-aging drug/supplement.

The yeast cells used in the above method may not express a functional telomerase or express telomerase components under an inducible promoter.

The substance effecting/having an effect on/influencing telomere length may also be designated as test substance. The method of the second aspect of the present invention can be carried out as high-throughput method allowing the analysis of two or more test substances in parallel, e.g. of 96 test substances in a 96-well-plate.

In a third aspect, the present invention relates to a yeast cell expressing a first telomere binding fusion protein comprising a bioluminescence donor and a second telomere binding fusion protein comprising a fluorescence acceptor. The yeast cell is preferably a recombinant/genetically modified yeast cell.

Particularly,
the first telomere binding fusion protein comprises Rif1 or Rif2 and a bioluminescence donor and the second telomere binding fusion protein comprises Rap1 and a fluorescence acceptor, or the first telomere binding fusion protein comprises Rap1 and a bioluminescence donor and the second telomere binding fusion protein comprises Rif1 or Rif2a and a fluorescence acceptor.

More particularly, the first telomere binding fusion protein comprises Rif2 and a bioluminescence donor and the second telomere binding fusion protein comprises Rap1 and a fluorescence acceptor.

Thus, in one preferred embodiment, the yeast cell expresses

a first telomere binding fusion protein comprising Rif2 and a bioluminescence donor, and
a second telomere binding fusion protein comprising Rap1 and a fluorescence acceptor.

Specifically, the (heterologous) nucleotide sequences encoding the bioluminescence donor and the fluores-cence acceptor are integrated into the yeast cell genome.
For example,

the (heterologous) nucleotide sequence encoding the bioluminescence donor is preferably cloned in-frame with (the nucleotide sequence encoding) Rif1, Rif2, or Rap1, and/or
the (heterologous) nucleotide sequence encoding the fluorescence acceptor is preferably cloned in-frame with (the nucleotide sequence encoding) Rif1, Rif2, or Rap1.

The (heterologous) nucleotide sequence encoding the bioluminescence donor may be cloned in-frame with (the nucleotide sequence encoding) Rif2 such that it is fused to the N-terminus and/or C-terminus of Rif2 in the final fusion protein. In addition, the (heterologous) nucleotide sequence encoding the fluorescence acceptor may be cloned in-frame with (the nucleotide sequence encoding) Rap1 such that it is fused to the N-terminus and/or C-terminus of Rap1 in the final fusion protein. More preferably, the (heterologous) nucleotide sequence encoding the bioluminescence donor is cloned in-frame with (the nucleotide sequence encoding) Rif2 such that it is fused to the N-terminus of Rif2 in the final fusion protein, and/or the (heterologous) nucleotide sequence encoding the fluorescence acceptor is cloned in-frame with (the nucleotide sequence encoding) Rap1 such that it is fused to the C-terminus of Rap1 in the final fusion protein.

Alternatively, the nucleotide sequences encoding the natural-occurring telomere binding protein, e.g. Rap1, Rif1, and/or Rif2, is replaced by a nucleotide sequence encoding a fusion protein composed of the telomere binding protein, e.g. Rap1, Rif1, and/or Rif2, and a bioluminescence donor or fluorescence acceptor in the yeast cell genome.
The bioluminescence donor may be localized/present at the N-terminus and/or C-terminus of Rif1 or Rif2. In addition, the fluorescence acceptor may be localized/present at the N-terminus and/or C-terminus of Rap1. Preferably, the biolumi-nescence donor is present at the N-terminus of Rif2, and/or the fluorescence acceptor is present at the C-terminus of Rap1.

Thus, in one more preferred embodiment, the yeast cell expresses

a first telomere binding fusion protein comprising Rif2 and a bioluminescence donator, and
a second telomere binding fusion protein comprising Rap1 and a fluorescence acceptor, wherein
the bioluminescence donator is fused to the N-terminus of Rif2, and/or
the fluorescence acceptor is fused to the C-terminus of Rap1.

It is preferred that the bioluminescence donator is a luciferase. It is more preferred that the luciferase is *Renilla reniforinis*-luciferase (RLuc). The *Renilla reniformis-luciferase* (RLuc) has preferably an amino acid sequence according to SEQ ID NO: 1.

It is further preferred that the fluorescence acceptor is a yellow fluorescent protein (YFP) or green fluorescent protein 2 (GFP²). It is more preferred that the yellow fluorescent protein (YFP) is an enhanced yellow fluorescent protein (EYFP) or a modified yellow fluorescent protein (YFP). The modified YFP has been mutated to have a higher affinity for luciferases (interacting YFP) as described in WO 2024/089297 (PCT/EP2023/080279). Specifically, the transient interaction of the mutated YFP and its higher quantum yield results in a stronger BRET signal at the same level of interaction between the proteins fused to donator and acceptor. This allows the detection of weaker interactions in a broader dynamic signal range. The modified YFP has preferably an amino acid sequence according to SEQ ID NO: 2. Thus, in one even more preferred embodiment, the yeast cell expresses a first telomere binding fusion protein comprising Rif2 and a luciferase such as a *Renilla reniformis-luciferase* (RLuc) as bioluminescence donator, and

a second telomere binding fusion protein comprising Rap1 and a yellow fluorescent protein (YFP) such as an enhanced yellow fluorescent protein (EYFP) or a modified yellow fluorescent protein (YFP) as fluorescence acceptor, wherein
the bioluminescence donator is fused to the N-terminus of Rif2, and/or
the fluorescence acceptor is fused to the C-terminus of Rap1.

The first telomere binding fusion protein comprising Rif2 and a *Renilla reniformis*-luciferase (RLuc) as bioluminescence donator has preferably an amino acid sequence according to SEQ ID NO: 3.

The second telomere binding fusion protein comprising Rap1 and modified YFP as fluorescence acceptor has preferably an amino acid sequence according to SEQ ID NO: 4.

Preferably, the yeast cell is selected from the genera *Saccharomyces, Rhodotorula, Schizosaccharomyces, Kluyveromyces, Pichia, Hansenula, Rhodosporidium, Candida, Yarrowia, Lipomyces* and *Cryptococcus.* More preferably, the yeast cell is a *Saccharomyces cerevisiae* cell.

Thus, in one still even more preferred embodiment, the *Saccharomyces cerevisiae* cell expresses

a first telomere binding fusion protein comprising Rif2 and a luciferase such as a *Renilla reniformis*-luciferase (RLuc) as bioluminescence donator, and
a second telomere binding fusion protein comprising Rap1 and a yellow fluorescent protein (YFP) such as an enhanced yellow fluorescent protein (EYFP) or a modified yellow fluorescent protein (YFP) as fluorescence acceptor, wherein
the bioluminescence donator is fused to the N-terminus of Rif2, and/or
the fluorescence acceptor is fused to the C-terminus of Rap1.

The yeast cells described above preferably does not express a functional telomerase or expresses telomerase components under an inducible promoter.

In one most preferred embodiment, the *Saccharomyces cerevisiae* cell is a cell deposited at the DSMZ with the deposit number DSM 35004. In particular, the *Saccharomyces cerevisiae* cell was deposited under the name "Telo View Lumi WT" with the DSMZ-Deutsche Sammlung von Mikroorganismen und Zellkulturen GmbH, Inhoffenstrasse 7B, 38124 Braunschweig, Germany on April 22, 2024 under deposit number DSM 35004.

In a fourth aspect, the present invention relates to the use of the yeast cell of the third aspect for determining telomere length.

As to preferred embodiments, it is referred to the first aspect of the present invention.

In a fifth aspect, the present invention relates to the use of the yeast cell of the third aspect for identifying a substance effecting/having an effect on/influencing telomere length.

Preferably, the substance effecting/having an effect on/influencing telomere length is selected from the group consisting of a drug, foodstuff, food supplement, secondary metabolite of an organism, and another chemical compound. More preferably, the drug is an anti-cancer drug, a growth factor, G-quadruplex stabiliser/destabiliser, senolytic, senostatic, senomorphic, or anti-aging drug/supplement.

As to preferred embodiments, it is referred to the second aspect of the present invention.

The invention is summarized as follows:

1. A method for determining telomere length comprising the steps of:

(i) providing yeast cells expressing

(ia) a first telomere binding fusion protein comprising a bioluminescence donator, and
(ib) a second telomere binding fusion protein comprising a fluorescence acceptor,

(ii) incubating the yeast cells provided in (i) with a bioluminescence donator substrate,
(iii) detecting bioluminescence of the bioluminescence donator at a wavelength characteristic for the donator and detecting fluorescence of the fluorescence acceptor at a wavelength characteristic for the acceptor (in case of telomeric binding of the first telomere binding fusion protein and the second telomere binding fusion protein in physical proximity), and
(iv) determining the acceptor/donator ratio at both wavelengths, wherein the ratio at said wavelengths is indicative for the telomere length.

2. The method of item 1, wherein the telomere length is calculated by correlating the ratio of step (iv) to a telomere length using a calibration curve.

3. A method for identifying a substance effecting/having an effect on/influencing telomere length comprising the steps of:

(i) providing yeast cells expressing

(ia) a first telomere binding fusion protein comprising a bioluminescence donator, and
(ib) a second telomere binding fusion protein comprising a fluorescence acceptor,

(ii) incubating the yeast cells provided in (i) with a bioluminescence donator substrate and a substance potentially effecting /having an effect on/influencing telomere length,
(iii) detecting bioluminescence of the bioluminescence donator at a wavelength characteristic for the donator and detecting fluorescence of the fluorescence acceptor at a wavelength characteristic for the acceptor (in case of telomeric binding of the first telomere binding fusion protein and the second telomere binding fusion protein in physical proximity),
(iv) determining the acceptor/donator ratio at both wavelengths, and
(v) comparing the acceptor/donator ratio determined in (iv) with an acceptor/donator reference ratio (obtained under the same conditions but without the substance potentially effecting/having an effect on/influencing telomere length), wherein a difference between the ratio and the reference ratio indicates that the substance effects/has an effect on/influences telomere length.

4. The method of any one of items 1 to 3, wherein

the first telomere binding fusion protein comprises Rap1-interacting factor 1 (Rifl) or Rap 1-interacting factor 2 (Rif2) and a bioluminescence donator and the second telomere binding fusion protein comprises Rap1 and a fluorescence acceptor, or
the first telomere binding fusion protein comprises Rap1 and a bioluminescence donator and the second telomere binding fusion protein comprises Rif1 or Rif2 and a fluorescence acceptor.

5. The method of item 4, wherein the first telomere binding fusion protein comprises Rif2 and a bioluminescence donator and the second telomere binding fusion protein comprises Rap1 and a fluorescence acceptor.

6. The method of any one of items 1 to 5, wherein the first telomere binding fusion protein and the second telomere binding fusion protein bind to double-stranded regions comprising telomeric DNA-repeat sequences.

7. The method of any one of items 4 to 6, wherein the (first/second) telomere binding fusion protein directly binds via Rap1 double-stranded regions with telomeric DNA-repeat sequences and wherein the (first/second) telomere binding fusion protein indirectly binds via Rif1 or Rif2 double-stranded regions with telomeric DNA-repeat sequences by association of Rif1 or Rif2 with Rap1.

8. The method of any one of items 1 to 7, wherein by telomeric binding of the first telomere binding fusion protein and the second telomere binding fusion protein in physical proximity a bioluminescence resonance energy transfer takes place so that bioluminescence of the bioluminescence donator as well as fluorescence of the fluorescence acceptor is detectable.

9. The method of any one of items 1 to 8, wherein (heterologous) nucleotide sequences encoding the bioluminescence donator and the fluorescence acceptor are integrated into the yeast cell genome.

10. The method of any one of items 4 to 9, wherein

the (heterologous) nucleotide sequence encoding the bioluminescence donator is cloned in-frame with Rif1, Rif2, or Rap1, and/or
the (heterologous) nucleotide sequence encoding the fluorescence acceptor is cloned in-frame with Rif1, Rif2, or Rap1.

11. The method of any one of items 4 to 10, wherein the bioluminescence donator is fused to the N-terminus and/or C-terminus of Rif2.

12. The method of item 11, wherein the bioluminescence donator is fused to the N-terminus of Rif2.

13. The method of any one of items 4 to 12, wherein the fluorescence acceptor is fused to the N-terminus and/or C-terminus of Rap1.

14. The method of item 13, wherein the fluorescence acceptor is fused to the C-terminus of Rap1.

15. The method of any one of items 1 to 14, wherein the bioluminescence donator is a luciferase.

16. The method of item 15, wherein the luciferase is *Renilla reniformis*-luciferase (RLuc).

17. The method of any one of items 1 to 16, wherein the fluorescence acceptor is a yellow fluorescent protein (YFP) or green fluorescent protein 2 (GFP$^2$).

18. The method of any one of items 1 to 17, wherein the bioluminescence donator substrate is coelenterazine or coelenterazine 400a (DeepBlue C).

19. The method of any one of items 1 to 18, wherein the yeast cells are *Saccharomyces cerevisiae* cells.

20. The method of any one of items 15 to 19, wherein the bioluminescence of luciferase is detected at a wavelength of between 430 and 485 nm, preferably at a wavelength of 481 nm.

21. The method of any one of items 17 to 20, wherein the fluorescence of YFP is detected at a wavelength of between 505 and 590 nm, preferably at a wavelength of 527 nm.

22. The method of any one of items 1 to 21, wherein the yeast cells have an optical density of between 0.1 and 0.9 at the time of adding the substrate.

23. The method of any one of items 1 to 22, wherein the yeast cells do not express a functional telomerase or express telomerase components under an inducible promoter.

24. The method of any one of items 3 to 23, wherein a ratio (acceptor/donator) above the reference ratio (acceptor/donator) indicates that the substance increases telomere length/positively effects telomer length.

25. The method of any one of items 3 to 23, wherein a ratio (acceptor/donator) below the reference ratio (acceptor/donator) indicates that the substance decreases telomere length/negatively effects telomer length.

26. The method of any one of items 3 to 25, wherein the substance is selected from the group consisting of a drug, foodstuff, food supplement, secondary metabolite of an organism, and another chemical compound.

27. The method of item 26, wherein the drug is an anti-cancer drug, a growth factor, G-quadruplex stabiliser/destabiliser, senolytic, senostatic, senomorphic or anti-aging drug/supplement.

28. A yeast cell expressing a first telomere binding fusion protein comprising a bioluminescence donator and a second telomere binding fusion protein comprising a fluorescence acceptor.

29. The yeast cell of item 28, wherein

the first telomere binding fusion protein comprises Rif1 or Rif2 and a bioluminescence donator and the second telomere binding fusion protein comprises Rap1 and a fluorescence acceptor, or
the first telomere binding fusion protein comprises Rap1 and a bioluminescence donator and the second telomere binding fusion protein comprises Rif1 or Rif2a and a fluorescence acceptor.

30. The yeast cell of item 29, wherein the first telomere binding fusion protein comprises Rif2 and a bioluminescence donator and the second telomere binding fusion protein comprises Rap1 and a fluorescence acceptor.

31. The yeast cell of any one of items 28 to 30, wherein the (heterologous) nucleotide sequences encoding the bioluminescence donator and the fluorescence acceptor are integrated into the yeast cell genome.

32. The yeast cell of any one of items 29 to 31, wherein

the nucleotide sequence encoding the bioluminescence donator is cloned in-frame with Rif1, Rif2, or Rap1, and/or
the nucleotide sequence encoding the fluorescence acceptor is cloned in-frame with Rif1, Rif2, or Rap1.

33. The yeast cell of any one of items 29 to 32, wherein the bioluminescence donator is fused to the N-terminus and/or C-terminus of Rif2.

34. The yeast cell of item 33, wherein the bioluminescence donor is fused to the N-terminus of Rif2.

35. The yeast cell of any one of items 29 to 34, wherein the fluorescence acceptor is fused to the N-terminus and/or C-terminus of Rap1.

36. The yeast cell of item 35, wherein the fluorescence acceptor is fused to the C-terminus of Rap1.

37. The yeast cell of any one of items 28 to 36, wherein the bioluminescence donor is a luciferase.

38. The yeast cell of item 37, wherein the luciferase is *Renilla reniformis-luciferase* (RLuc).

39. The yeast cell of any one of items 28 to 38, wherein the fluorescence acceptor is a yellow fluorescent protein (YFP) or green fluorescent protein 2 (GFP[2]).

40. The yeast cell of any one of items 28 to 39, wherein the yeast cell is a *Saccharomyces cerevisiae* cell.

41. The yeast cell of any one of items 28 to 40, wherein the yeast cell does not express a functional telomerase.

42. Use of the yeast cell of any one of items 28 to 41 for determining telomere length.

43. Use of the yeast cell of any one of items 28 to 41 for identifying a substance effecting/having an effect on/influencing telomere length.

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

BRIEF DESCRIPTION OF THE FIGURES

The following Figures are merely illustrative of the present invention and should not be construed to limit the scope of the invention as indicated by the appended claims in any way.

**FIGURE 1:** Shows a schematic representation of bioluminescence resonance energy transfer (BRET). Protein A fused to Rluc and Protein B fused to EYFP. Protein A = Rif1 or Rif2, Protein B = Rap1, EYFP = enhanced yellow fluorescent protein (EYFP), Rluc = *Renilla reniformis*-luciferase. Only in case proteins Rif1 or Rif2 and Rap1 are in physical proximity on a telomeric sequence, energy generated from a luciferase enzyme as bioluminescence donor excites EYFP as fluorescence acceptor. The energy emitted by the acceptor relative to the energy emitted by the donor is called the BRET signal or BRET ratio. It depends on the spectral properties, the ratio, distance and relative orientation of the donor and acceptor molecules as well as the strength and stability of the interaction between the proteins of interest.

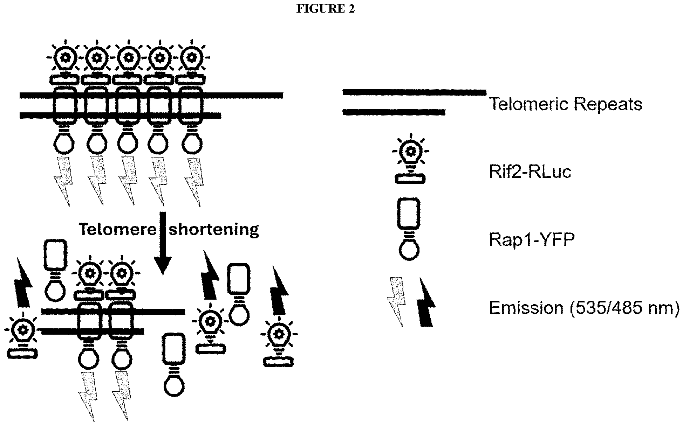

**FIGURE 2:** Shows a schematic summary of the invention's main idea.

Small boxes - Rif2
Big boxes - Rap1
Shining lightbulbs with internal gear - Rluc with substrate
Non-shining lightbulbs YFP
Lightning - Emission (black from RLuc and grey from YFP)
Only in case proteins Rif1 or Rif2 and Rap1 are in physical proximity on a telomeric sequence, energy generated from a luciferase enzyme as bioluminescence donor excites YFP as fluorescence acceptor. The energy emitted by the acceptor relative to the energy emitted by the donor is called the BRET signal or BRET ratio.
The BRET ratio increases with increasing telomere length as it increases the number of binding sites for the telomere binding protein Rap1-RLuc, which only in the bound state forms a protein complex with Rif1-YFP.

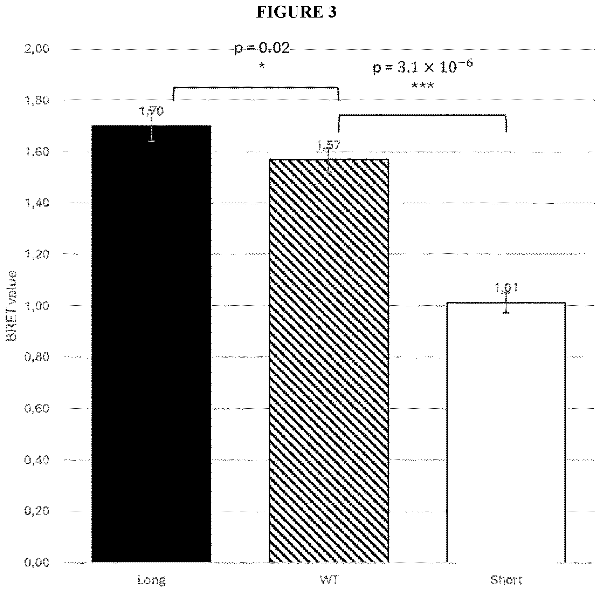

**FIGURE 3:** Shows the exemplified results of a measurement of BRET values measured from the strains WT (wild type, regular telomere length), Long (*Δelgl1*, elongated telomeres), Short (*Δtel1*, decreased telomere length), respectively (see description in the chapter "Final Strains"). All measurements were performed using technical independent triplicates.

BRET values were calculated as follows:

$$\mathrm{BRET} = I_A/I_D - c_f$$

$I_A$ Luminescence intensity [Counts/s] for acceptor (505 nm to 590 nm)
$I_D$ Luminescence intensity [Counts/s] for donor (430 nm to 485 nm)
$c_f$ Control factor = $I_A/I_D$ for C- strain measured in the same experiment

Error bars represent standard deviation of BRET values.

Statistical significance and p values were determined by two-sided two-sample t-test.

The BRET values of the three strains correlate with their telomere length.

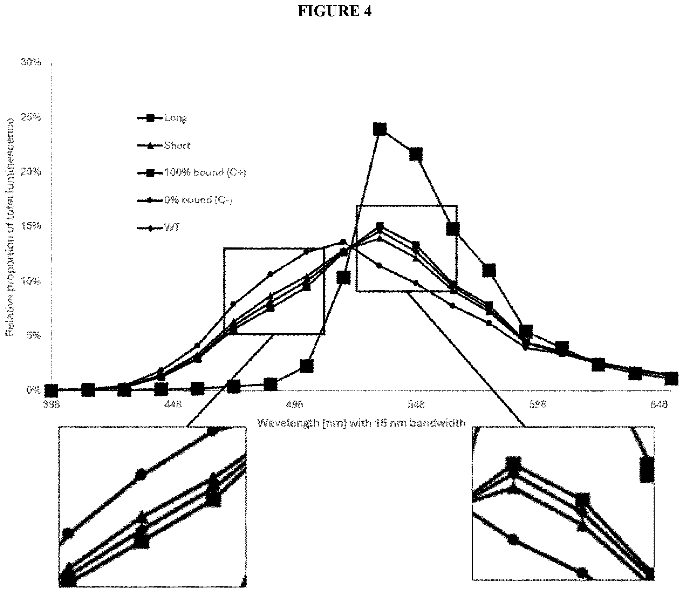

**FIGURE 4:** Shows the luminescence spectra measured from the strains WT (wild type, regular telomere length, diamonds as symbol), Long (*Δelgl1,* elongated telomeres, small boxes as symbol), Short (*Δtel1*, decreased telomere length, triangles as symbol), C+ (fusion protein of donor and acceptor, giving a maximum BRET signal, larger boxes as symbol) and C- (only donor, no acceptor, no BRET signal, dots as symbol) as explained in the chapter "Final Strains".

The difference of the signals between C- and C+ are caused by a shift from lower to higher wavelength which illustrates the difference between the pure donor (RLuc) signal and the maximum bioluminescence energy transfer (BRET) to the acceptor (YFP) with almost no donor signal left.

In this order the strains Long, wild type (WT) and Short show a decreased rate of transfer between donor and acceptor which is coherent with their respective telomere lengths.

The areas of the spectra that were used to calculate BRET values are marked and magnified in order to highlight the differences.

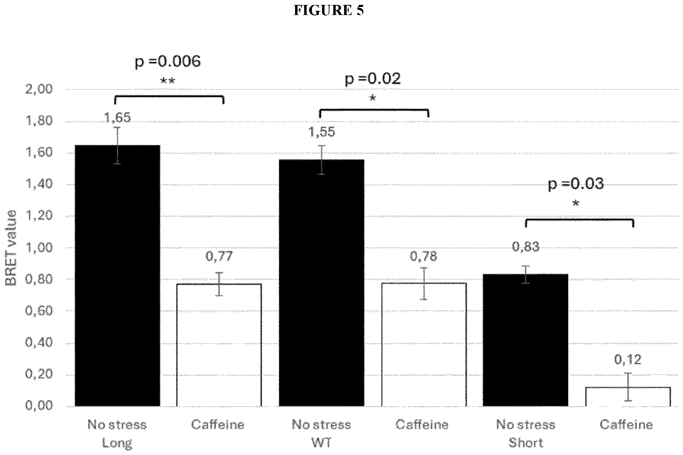

**FIGURE 5:** Shows the results of a measurement of BRET values for an experiment testing the effect of caffeine as chemical stressors on the telomere length of the yeast cells. The strains used in the experiment are Long (*Δelgl1,* elongated telomeres), WT (wildtype, regular telomere length) and Short (*Δtell1*, shortened telomeres)

BRET values were calculated as follows:

$$\mathrm{BRET} = I_A/I_D - c_f$$

$I_A$ Luminescence intensity [Counts/s] for acceptor (505 nm to 590 nm)

$I_D$ Luminescence intensity [Counts/s] for donor (430 nm to 485 nm)

$c_f$ Control factor = $I_A/I_D$ for C- strain measured in the same plate

Error bars represent standard deviation of BRET values.

Statistical significance and p values were determined by two-sided two-sample t-test.

The figure shows that caffeine negatively influences telomere length in a statistically significant manner.

EXAMPLES

The examples given below are for illustrative purposes only and do not limit the invention described above in any way.

EXAMPLE 1

Materials & Methods, Strains

As yeast strains the *Saccharomyces cerevisiae* strains BY4741/BY4742 and as indicated knock-out strains based on these strains obtained from EUROSCARF were used.

*Escherichia coli* DH5α was used as a bacterial cloning strain.

Cultivation

If not stated otherwise, yeast was grown on Yeast Peptone Dextrose (YPD) liquid medium or agar plates prepared as described before. Bacto Yeast Extract, Peptone and Agar were obtained from BD Biosciences, New Jersey and Glucose from Carl Roth, Karlsruhe.

Drop-out essays were performed on Standard Defined (SD) plates prepared with Difco Yeast Nitrogen Base without Amino Acids (BD Biosciences, New Jersey), 2 % Glucose and the necessary supplements.

Yeast on solid media was incubated at 30°C in 90 mm petri dishes (LABSOLUTE, Renningen). Liquid yeast cultures were incubated at 30°C by shaking at 180 rpm (New Brunswick Eppendorf Innova) in 3 ml culture volume in 100 mm glass culture tubes or in 50 ml culture volume in 100 ml Erlenmeyer culture flasks (BRAND, Wertheim).

Bacterial cultures for plasmid propagation were grown on Luria Broth medium containing 1 % Bacto Tryptone (BD Biosciences, New Jersey), 1 % NaCl (Th. Geyer, Renningen) and 0.5 % Bacto Yeast Extract.

Plasmid selection was done by supplementing the media with 100 μg/ml Ampicillin (AppliChem, Darmstadt) or 50 μg/ml Kanamycin (Carl Roth, Karlsruhe).

Vectors

Vectors for CRISPR/Cas9 (sgRNA entry vector pWS082 and Cas9 expression vector pWS158) were a gift from Tom Ellis (Addgene plasmid # 90516; http://n2t.net/addgene:90516; RRID:Addgene_90516) and used according to the detailed protocol of the developing lab.

The protocol describes the insertion of sgRNA sequence oligos by golden gate assembly and the preparation of linearized expression and entry vectors for transformation.

The vector pDUD with the DDI2 promoter was a gift from Wei Xiao (University of Saskatchewan).

The vectors for amplifying the tags (pJU7410 for RLuc and pJU7426 for modified YFP) were a gift from Jörg Urban (patent application WO 2024/089297 (PCT/EP2023/080279)).

Transformation

Transformation of yeast was either carried out using the lithium acetate method or using the Frozen-EZ Yeast Transformation II kit (ZYMO RESEARCH, Orange) according to the company protocol.

After the transformation cells were plated on SD plates selecting for the auxotrophic marker on the CRISPR/Cas9 plasmid and grown for up to one week either on 30 °C or room temperature until visible colonies were formed.

For the marker-free genomic modification of yeast the following DNA mix was used for transformation as described: 100 ng pWS158 (Cas9), 200 ng pWS082 (sgRNA entry vector) distributed on the number of sgRNAs used (e.g. for 2 modifications with 2 sgRNAs each 50 ng of each sgRNA vector), 10-15 μl of fresh PCR product (unpurified, at least 5 μg).

Competent DH5α were transformed using the calcium chloride method.

Design of constructs

For each knock-in a panel of 10 DNA oligos were designed:

1) 2 sgRNA oligos with linker sequences for the sgRNA entry vector to be annealed and inserted.
2) 6 primers for overlap extension PCR to build the homologous repair construct that integrates into the genome. Alternatively, 2 primers including a long homology stretch to the targeted region to amplify the insert with.
3) 2 primers to verify insertion of the knock-in from upstream or downstream (or both to be sure of the correct insertion) by colony PCR

sgRNA design

CRISPR/Cas9 target sequences were selected with CCTop which offers efficiency scores of targets and checks for target specificity in many species including *S. cerevisiae*.

Using one or multiple viable target sequences, design of oligos was completed and annealing and cloning into the entry vector was performed as explained in the protocol.

For the promoter shuffle of *EST2* two target sites were chosen: 99 and 121 bp upstream of the *EST2* start codon (GAATGAAAAAAAGTAAAGCA (SEQ ID NO: 5) and GCTATTGGTGATTCGCATTT (SEQ ID NO: 6)).

For the C-terminal fusion of YFP to Rap1 150 bp including the *RAP1* stop codon were exchanged for the YFP-knock-in without regard to putative terminator sequences since the inserted YFP gene included the *ADH1* terminator sequence. The chosen target sequences (TTATTTCACTGATTCACGTT (SEQ ID NO: 7) and AATTCTTACAATGATTA AGG (SEQ ID NO: 8)) are 44 and 127 bp downstream of the *RAP1* stop codon.

For N-terminal fusion of RLuc to Rif2 a target 19 bp downstream of the start codon was used (TCTCCTTA TAGGTGCAAAAT (SEQ ID NO: 9)). To remove the target site by the knock-in, the recombination cassette changed the codon of Aspartic Acid 7 of *RIF2* from GAT to GAC, which changes the second base of the sgRNA target sequence after the PAM.

Recombination cassette design

The recombination cassettes used here are linear DNA fragments produced by PCR. The 5' and 3' ends of the PCR product consist of sequences homolog to the genomic target sequences. Homolog sequences had a length between 92 and 950 bp (see primer sequences).

The part between the homolog sequences is the insert (YFP/RLuc/pDDI).

The cassette is constructed by either amplification of the insert with primers including 100 bp of homolog sequence on their 5' ends or by Overlap Extension PCR (OEPCR) were the 5' part is one primer for amplification of the homolog sequences. For OEPCR the reverse complement of these primers and a pair of outer primers at the up- and downstream ends of the homolog sequence were designed.

For the N-terminal tagging of Rif2 with RLuc the primers were:

- Outer forward primer CGATCCGAAGCTACTTATGC (SEQ ID NO: 10) 525 bp upstream of the RIF2 start codon
- Outer reverse primer GTTGTCCGCTATATGAAGCC (SEQ ID NO: 11) 498 bp downstream from the RIF2 start codon
- Inner forward primer CGAACGTGGTTAGTATATAGAGACAATGACTCAATTGAACGGATCTG (SEQ ID NO: 12) last 25 bp before the RIF2 start codon and first 22 bp including the start codon of the modified RLuc on the plasmid pJU7410
- Inner reverse primer GCAAAGTCGGAATCTACATGCTCCATTTTAGAAGATCTGTTACCACTACCG (SEQ ID NO: 13) first 26 bp including the start codon of RIF2 with the silent mutation (A to G) in D7 as mismatch and the last 25 bp before the stop codon of RLuc on the plasmid pJU7410
- The reverse complements of the inner primers CAGATCCGTTCAATTGAGTCATTGTCTCTATATACTAACCACGTTCG (SEQ ID NO: 14) and
- CGGTAGTGGTAACAGATCTTCTAAAATGGAGCATGTAGATTCCGACTTTGC (SEQ ID NO: 15)
- Validation forward primer CTAACGGTGATCCGCGGAG (SEQ ID NO: 16) at the end of the RLuc insert 34 bp before the RIF2 start codon
- Validation reverse primer CCATCATCATTAGGTCAGACGTGTG (SEQ ID NO: 17) 32 bp after the downstream homology of the integration cassette within the coding region of RIF2

For the C-terminal tagging of RAP1 with modified YFP the primers are:

- Outer forward primer CAATAGACCCAAGAGGCCTG (SEQ ID NO: 18) 727 bp upstream of the RAP1 stop codon
- Outer reverse primer CTTCCAGCAAATCTAACAGCATC (SEQ ID NO: 19) 948 bp downstream from the RAP1 stop codon
- Inner forward primer GAAAAGATTTTTTGAGAAGGACCTGTTAATGACTCAATTGAACGGATCTG (SEQ ID NO: 20) last 28 bp before the RAP1 stop codon (exuding it) the and first 22 bp including the start codon of the modified YFP on the plasmid pJU7426
- Inner reverse primer GCTAATGGGATTCTATAAAACTGTTCCGGTACCATTACCCTGTTATCCC (SEQ ID NO: 21) first 28 bp after the second selected Cas9 target 157 bp after the RAP1 stop codon and the last 21 bp of the terminator sequence on the plasmid pJU7426
- The reverse complements of the inner primers CAGATCCGTTCAATTGAGTCATTAACAGGTCCTTCTCAAAAAATCTTTTC (SEQ ID NO: 22) and
- GGGATAACAGGGTAATGGTACCGGAACAGTTTTATAGAATCCCATTAGC (SEQ ID NO: 23)
- Validation forward primer CTTATTGACCACACCTCTACCGG (SEQ ID NO: 24) within the terminator sequence 185 bp after the stop codon of the integrated YPF
- Validation reverse primer CTGTTAATTTCTTTTTAGACCCACCA (SEQ ID NO: 25) 1 bp after the downstream homology of the integration cassette

For promotor shuffle long primers instead of OEPCR were used:

- Forward primer GAAGCCGATTTTCTATTATCCCGTTCTGATAATCGATTTTTAGCGGATTATT AACTTTCTTAACC ATAACTAACACGCCCTCATGAATATTATCCTCTAAGAT AAAACACAGATCGACAG (SEQ ID NO: 26) 95 bp homology of the upstream region of the EST2 gene starting 154 bp upstream of the ETS2 start codon and first 24 bp of the DDI2 promotor on the pDUD plasmid
- Reverse primer AAGTGACCACATTTTAAATTTTCTTTGTAAGTACTGTTGGTCTGTAGATCA ATGTCAAGCTTGT CTTGAATGAACTCGAATAAGATTTTCATGATTGATTCT TTTGAAGAGAAGCAAGGC (SEQ ID NO: 27) 92 bp homology of the of the EST2 gene starting with the ETS2 start codon and last 28 bp of the DDI2 promotor on the pDUD plasmid
- Validation forward primer TCTAAGATAAAACACAGATCGAC (SEQ ID NO: 28) first 23 bp of DDI2 promotor
- Validation reverse primer CCAGGAAGGCATGGTAATGC (SEQ ID NO: 29) 58 bp after the homology of the integration cassette within the coding region of EST2

PCR

All preparative PCR (producing fragments for cloning or transformation) was carried out with KAPA HiFi PCR Kit (Roche Diagnostics, Mannheim) with the included reagents according to the manual.

Colony PCR to validate integration was carried out with DreamTaq DNA Polymerase (ThermoFisher, Darmstadt) with the included reagents according to the manual.

The OEPCR is done in two steps. First the two homology fragments and the insert fragment were amplified separately. The primers were outer forward/inner forward reverse complement primers (upstream homology), inner reverse complement/outer reverse primers (downstream homology) and inner forward/ inner reverse primers (insert). The fragments were separated by agarose gel electrophoresis, excised and purified from the gel with a NucleoSpin kit (Macherey Nagel, Allentown).

Second the three fragments were mixed in an equimolar ratio (100 ng of the largest fragment) and passed PCR for 10 - 15 cycles without primers. After that the outer primers were added and another 25 to 30 cycles of PCR were performed.

Program: 95 °C 3 min., (98 °C 20 sec., 72 °C 40 sec.) x15, 72 °C 1 min., hold 72 °C, add outer primers, 98 °C 20 sec. (98 °C 20 sec., 58 °C 15 sec., 72 °C 70 sec.) x30, 72 °C 3 min.

The full length fragment was purified from agarose gel.

Final Strains

- WT is wildtype in BY4741 (*his3Δ1 leu2Δ0 met15Δ0 ura3Δ0,* Mating Type a) with the two modifications RIF2::RLuc (N-terminal) and RAP1::modified YFP (c-terminal).
- C- is a negative control in BY4741 (*his3Δ1 leu2Δ0 met15Δ0 ura3Δ0,* Mating Type a) with only the donor RIF2::RLuc (N-terminal) giving the 0 % interaction BRET background.
- C+ is a positive control in BY4741 (*his3Δ1 leu2Δ0 met15Δ0 ura3Δ0,* Mating Type a) with a donor-acceptor fusion protein RIF2:: modified YFP-RLuc (N-terminal) giving the 100 % interaction maximum BRET-signal
- Short is a *Δtel1* knock-out strain in BY4742 background (*leu2Δ0 lys2Δ0 ura3Δ0,* Mating Type α). A knock out of the gene TEL1 has been shown to greatly reduce telomere length compared to wildtype [1].
- Long is a *Δelgl1* knock-out strain in BY4742 background (*leu2Δ0 lys2Δ0 ura3Δ0,* Mating Type α). A knock out of the gene TEL1 has been shown to greatly increase telomere length compared to wildtype [1].

All colonies chosen for further work were checked for correct auxotrophy markers and were analyzed by colony PCR and phenotypically by luminescence and fluorescence.

BRET measurements

Cultures were grown at 30°C in YPD overnight.

Cultures were diluted to an $OD_{600}$ of 0.3 using YPD in a white flat bottom 96-well plate (Microplate 96/F-PP, Eppendorf, Hamburg) for measurement.

Native Coelenterazine (Carl Roth, Karlsruhe) was solved at 1 mg/ml (2.361 µM) in ethanol (>99.5 %, Carl Roth) + 3 mM HCl (6 M, Carl Roth). The stocks were stored in aliquots of 200 µl in dark glass vials (LABSOLUTE, Renningen) at -80 °C.

Between 15 and 60 minutes before measurement the Coelenterazine stock was diluted 1:94.4 to a concentration of 25 mM in YPD (= 5x substrate). 50 µl of the 5x substrate were added to the 200 µl cell suspension to a final concentration of 5 mM Coelenterazine for measurement. The multimode reader (Spark, Tecan, Männedorf) was prewarmed to 30 °C. The measurement program was:

1. Dosing 50 µl of 5x substrate to each well to be measured with the multimode reader's injector at 100 µl/s
2. 3x 5 minutes double orbital shaking, 6 mm amplitude at 54 rpm with 1 minute breaks
3. Measurement for each well of luminescence at 430 nm to 485 nm (donator) and 505 nm to 590 nm (acceptor) with 1 second integration time

Luminescence spectra were acquired after the measurement for each well at 398, 413, 428, 443, 458, 473, 488, 503, 518, 533, 548, 563, 578, 593, 608, 623, 638 and 653 nm with 15 nm peak width.

BRET values were calculated as follows:

$$BRET = I_A/I_D - c_f$$

$I_A$ Luminescence intensity [Counts/s] for acceptor (505 nm to 590 nm)
$I_D$ Luminescence intensity [Counts/s] for donator (430 nm to 485 nm)
cf Control factor = $I_A/I_D$ for C- strain measured in the same plate

Stress influence on telomere length

Yeast was grown in 50 ml YPD without additives (no stress) or with 10 mM caffeine ($\geq$98,5 %, Carl Roth) for 48 h with dilution in fresh media to $OD_{600}$ $4*10^{-3}$ after 24h.
Cells were measured as explained above.

Results

In order to measure telomere length in an easy, efficient and high-throughput capable manner, a system using BRET as a read-out was designed. The principle of the measuring is based on the observation that when telomeric binding proteins Rif1 or Rif2 and Rap1 come in physical proximity at a telomeric sequence, energy generated from a luciferase enzyme as bioluminescence donor excites YFP as fluorescence acceptor (**Figures 1** and **2**).
**Figure 3** demonstrates that yeast strains possessing different telomere lengths (wild type, *Δtel1* = Short telomeres, *Δelg1* = Long telomeres [1]) show BRET values corresponding to their telomere length. This shows that different telomere lengths can be detected by our system.
In **Figure 4** the luminescence spectra of control cells harboring only donator (C-, no BRET signal) or a donator/acceptor fusion protein (C+, maximum BRET signal) and the cells harboring wild type, short (*Δtel1*) and long (*Δetg1)* telomeres visualize the shift to longer wavelength through bioluminescence energy transfer (BRET). The wide emission spectrum of RLuc seen in C- is changed by maximum BRET (C+) to a narrow peak at longer wavelengths around 530 nm with almost no remaining emission in the lower wavelengths spectrum of RLuc. Whereas C+ and C- show the spectra of full and no BRET, the spectra of the mutant and wild type strains were localized in between. This shows the longer the telomeres of the strain *(Δtel1<WT<Δelg1)* the more similar the spectrum is to C+ and the less similar it is to C-.
To show that not only differences in telomere length caused by genetic factors can be measured and to demonstrate the potential of our system to be used for compound screens, we treated yeast cells with a chemical stressor and compared the result with untreated cells. Caffeine is a stressor known to negatively affect telomere length [2]. **Figure 5** shows that caffeine reduced the cells' telomere length compared to untreated cells. After two days of treatment with caffeine a statistically significant reduction of BRET ratio is visible in comparison to untreated cells. The effect is also detectable for mutant cells harboring long (*Δelg1*) or short (*Δtel1*) telomeres as described in the literature [6].
This demonstrates that the method is suitable for screening compounds that affect telomere length.

SHORT SUMMARY OF THE SEQUENCES

*Renilla reniformis-luciferase* (RLuc)

SEQ ID NO: 1

ATGACTCAATTGAACGGATCTGCTAGCGGAGGTTCTGGCGCGCCAACCGGAAATG
TGGCTTCTAAGGTTTACGACCCAGAACAAAGAAAAGGATGATTACTGGTCCTCA
ATGGTGGGCTAGATGCAAGCAAATGAATGTCTTAGATTCTTTCATCAACTACTAT
GACTCAGAGAAGCACGCTGAAAACGCAGTTATTTTTTTGCACGGTAACGCTGCAT
CCTCTTACTTATGGAGGCACGTCGTACCTCATATCGAGCCAGTTGCCAGATGTATC
ATTCCAGATTTGATTGGTATGGGTAAGTCTGGTAAATCAGGTAATGGCTCCTATA
GACTATTGGACCACTACAAGTACCTAACTGCTTGGTTTGAATTGCTAAACTTACC
AAAGAAATCATTTTCGTGGGACATGATTGGGGTTCTTGTTTGGCCTTTCATTATT
CTTACGAACATCAAGATAAGATCAAGGCTATTGTTCATGCGGAATCTGTTGTGGA
TGTTATTGAATCTTGGGAGGATTGGCCTGACATTGAAGAGGATATTGCTTTGTTA
AAGTCTGAAGAGGGTGAAAAATGGTGTTAGAAAATAACTTTTCGTTGAGACTA

TGTTACCATCAAAGATAATGAGAAAATTGGAACCTGAAGAGTTTGCTGCTTATTT
GGAACCATTCAAGGAAAAAGGCGAGGTTAGAAGGCCCACCCTATCTTGGCCTAG
AGAAATCCCATTAGTCAAAGGAGGCAAGCCAGATGTTGTCCAGATTGTTAGAAA
CTACAATGCCTATTTAAGGGCTTCTGACGATTTACCTAAGATGTTCATTGAATCCG
ACCCTGGTTTCTTTTCCAATGCCATTGTCGAAGGTGCTAAGAAATTCCCTAATACC
GAGTTTGTTAAGGTTAAGGGTTTGCACTTTTCTCAAGAGGATGCTCCAGACGAAA
TGGGTAAGTACATTAAATCCTTCGTGGAAAGGGTTTTGAAAAACGAACAGACCGC
TAACGGTGATCCGCGGAGTGGAGGAACCGGTAGTGGTAACAGATCTTCTAAA

<u>modified yellow fluorescent protein (YFP)</u>
SEQ ID NO: 2

ATGACTCAATTGAACGGATCTGCTAGCGGAGGTTCTGGCGCGCCAACCGGAAATG
TGTCCAAGGGTGAAGAATTGTTCACTGGTGTTGTCCCAATTTTGGTTGAATTAGAT
GGTGATGTTAATGGGCACAAATTTTCTGTCAGAGGAGAGGGTGAAGGTGATGCA
ACAATCGGTAAGTTGACCTTGAAAGTCATTTGCACTACTGGTAAACTACCTGTTC
CTTGGCCTACCTTGGTCACTACTGTTGGTTATGGTGTTCAATGCTTTGCTAGATAC
CCAGATCACATGAAGCAACATGACTTTTACAAGAGTGCCATGCCAGAAGGTTATG
TTCAGGAAAGAACTATCTCCTTCAAAGATGACGGTAAGTACAAGACTCGTGCTGA
AGTCAAGTTTGAAGGTGATACCTTGGTTAATAGAATCGAGTTAAAGGGTATTGAT
TTTAAGGAAGATGGTAACATTTTAGGTCACAAATTGGCTTACAACTTTAACTCTC
ACAATGTTTACATCACTGCTGACAAGCAAAAGAACGGTATCAAGGCTAACTTCAA
AATTAGACACAACATTGAGGATGGTGGTGTTCAACTAGCTGACCATTATCAACAA
AATACTCCAATCGGCGATGGCCCTGTCCTTTTACCAGACAACCATTACCTGTCCTA
CCAATCTCGTGTTTACAAAGATCCAAACGAAAAGAGAGACCACATGGCTGTTATT
GAGTTTATGACTGCTGCTGGAATTACTCATGGCATGGATGAACTACCGCGGAGTG
GAGGAACCGGTAGTGGTAACAGATCTTCTAAATAA

first telomere binding fusion protein RLuc-Rif2
SEQ ID NO: 3

ATGGAGCATGTAGATTCCGATTTTGCACCTATAAGGAGATCGAAAAAGGTTGTTG
ACAGTGACAAGATTGTGAAAGCAATAAGCGATGATTTGGAGCAAAAAAATTTTA
CTGTACTGAGAAAGTTGAACCTTGTTCCAATTAAAAAAAGTGTTAGCAGCCCAAA
GGTGTGTAAGCCGAGTCCAGTTAAGGAGCGAGTGGACCATGTTTTCTACCAGAAG
TTCAAATCAATGGCCTTGCAAGAGCTAGGCACCAATTATTTGTCAATAAGCTACG
TTCCGAGCTTAAGTAAGTTTCTTTCAAAAAATCTTAGGAGTATGAAAAATTGTAT
CGTTTTCTTCGACAAAGTTGAACATATACCAATATGCTGGTATCGACCGTGCA

GTTTCAGAAACACTGTCTTTAGTCGATATAAATGTCGTAATTATAGAAATGAATG
ACTACTTAATGAAAGAGGGTATTCAATCAAGCAAATCAAAGAATGTATCGAGT
CAATGGGGCAGGCTTCATATAGCGGACAACTAGATTTCGAAGCTAGTGAAAAAC
CTTCAAATCACACGTCTGACCTAATGATGATGGTTATGAGGAAAATAAATAATGA
CGAAAGTATCGATCATATTGTCTACTTCAAATTCGAACAATTAGATAAATTATCT
ACTTCAACTATAATAGAACCTTCGAAGCTTACCGAATTTATCAATGTTTTATCGGT
ACTTGAAAAAGTAATAACATTGCATTTAAGGTCCTCATTTATTCAAATAACGTT
AGCATTTCGTCTCTCCTATCGACATCCCTCAAAAAGAAACTCAACACAAATATA
CTGTGTTTGAGATGCCGATATTAACATGCGCTCAAGAACAAGAATATTTGAAAAA
AATGATAAAGTTTACCTTTGATTCCGGAAGCAAGTTATTACAGTCTTACAACTCG
CTTGTCACATGCCAGTTGAATAATAAAGAATCCAACTTAGCAATCTTTTTCGAATT
TTTGAAGGTCTTTCCGCACCCTTTTACCTATTTGTTTAACGCTTACACTGAGATTAT
AGTCCAGAGTAGAACTTTTGATGAATTGTTGGATAAGATCAGAAACAGACTGACA
ATAAAAAATTACCCACATAGTGCTTATAACTTTAAGAAAAACCAGCGTCTTCCAC
TTAAGTTAACTCGAAAAGTACATGATAGAATGACTCAATTGAACGGATCTGCTAG
CGGAGGTTCTGGCGCGCCAACCGGAAATGTGGCTTCTAAGGTTTACGACCCAGAA
CAAAGAAAAAGGATGATTACTGGTCCTCAATGGTGGGCTAGATGCAAGCAAATG
AATGTCTTAGATTCTTTCATCAACTACTATGACTCAGAGAAGCACGCTGAAAACG
CAGTTATTTTTTTGCACGGTAACGCTGCATCCTCTTACTTATGGAGGCACGTCGTA
CCTCATATCGAGCCAGTTGCCAGATGTATCATTCCAGATTTGATTGGTATGGGTA
AGTCTGGTAAATCAGGTAATGGCTCCTATAGACTATTGGACCACTACAAGTACCT
AACTGCTTGGTTTGAATTGCTAAACTTACCAAAGAAAATCATTTTCGTGGGACAT
GATTGGGGTTCTTGTTTGGCCTTTCATTATTCTTACGAACATCAAGATAAGATCAA
GGCTATTGTTCATGCGGAATCTGTTGTGGATGTTATTGAATCTTGGGAGGATTGGC
CTGACATTGAAGAGGATATTGCTTTGTTAAAGTCTGAAGAGGGTGAAAAAATGGT
GTTAGAAATAACTTTTTCGTTGAGACTATGTTACCATCAAAGATAATGAGAAAA
TTGGAACCTGAAGAGTTTGCTGCTTATTGGAACCATTCAAGGAAAAGGCGAGG
TTAGAAGGCCCACCCTATCTTGGCCTAGAGAAATCCCATTAGTCAAAGGAGGCAA
GCCAGATGTTGTCCAGATTGTTAGAAACTACAATGCCTATTTAAGGGCTTCTGAC
GATTTACCTAAGATGTTCATTGAATCCGACCCTGGTTTCTTTTCCAATGCCATTGT
CGAAGGTGCTAAGAAATTCCCTAATACCGAGTTTGTTAAGGTTAAGGGTTTGCAC
TTTTCTCAAGAGGATGCTCCAGACGAAATGGGTAAGTACATTAAATCCTTCGTGG
AAAGGGTTTTGAAAAACGAACAGACCGCTAACGGTGATCCGCGGAGTGGAGGAA
CCGGTAGTGGTAACAGATCTTCTAAATAA

second telomere binding fusion protein Rap1-modified YFP
SEQ ID NO: 4

ATGTCTAGTCCAGATGATTTTGAAACTGCACCAGCAGAATATGTTGATGCATTGG
ATCCGAGTATGGTCGTTGTTGACAGTGGTTCTGCTGCAGTCACAGCGCCATCGGA
TTCAGCCGCTGAGGTAAAAGCTAATCAAAACGAAGAAACACTGGTGCTACTGC
TGCTGAAACTAGTGAGAAAGTGGATCAGACGGAGGTCGAAAAGAAAGATGATGA
TGATACCACTGAAGTCGGTGTAACAACTACCACGCCTTCCATTGCTGATACTGCT
GCTACCGCTAACATTGCTTCGACATCAGGTGCATCTGTTACTGAACCTACTACTGA
TGACACCGCTGCTGATGAGAAAAAGGAACAAGTAAGTGGTCCTCCTCTGTCAAAT
ATGAAATTCTATCTTAATCGCGACGCGGATGCGCATGACTCTTTAAATGATATTG
ATCAATTAGCGCGTCTGATTAGAGCAAACGGTGGTGAAGTGCTAGACTCCAAGCC
AAGAGAATCAAAAGAGAATGTTTTCATCGTGTCACCCTATAATCATACCAATTTA
CCAACGGTAACTCCGACTTATATTAAGGCATGTTGTCAAAGCAACTCGCTACTGA
ACATGGAAAATTATTTAGTACCATACGATAACTTTAGAGAAGTTGTCGATAGTAG
GTTGCAGGAGGAATCGCATTCTAATGGTGTAGATAATAGCAATAGCAATAGCGAT
AACAAGGATTCTATCAGGCCCAAAACAGAAATAATCTACGAATACTAATGGC
GCTACGGAAGACTCAACCAGCGAAAAGTTATGGTAGACGCTGAACAACAAGCT
AGATTACAAGAGCAAGCTCAGCTCCTTCGTCAACATGTAAGCAGCACCGCATCAA
TCACAAGCGGAGGGCACAATGACTTGGTCCAGATCGAACAACCTCAAAAAGATA
CCTCCAATAATAATAATAGCAACGTCAACGACGAAGACAATGATCTTTTGACACA
GGACAACAACCCTCAAACAGCAGATGAGGGGAATGCATCTTTTCAAGCACAAAG
GTCCATGATTTCGAGAGGCGCTTTGCCCTCCCACAATAAAGCTTCTTTTACAGATG
AGGAAGATGAGTTTATTTTGGATGTTGTGAGAAAAAATCCAACCAGGCGTACAAC
ACATACTCTTTACGATGAAATATCCCATTATGTGCCTAACCACACGGGTAATTCTA
TTAGGCACCGATTTAGAGTCTATCTTTCCAAAAGACTAGAGTACGTTTATGAGGT
TGACAAGTTTGGTAAATTGGTTAGAGACGACGATGGAAATCTGATAAAGACTAA
AGTTTTGCCACCATCAATTAAAAGGAAATTTTCAGCAGATGAAGATTACACTTTG
GCAATCGCGGTCAAGAAGCAGTTTTATCGTGATTTGTTTCAAATCGACCCTGATA
CTGGAAGATCACTTATCACAGATGAGGATACACCCACTGCTATAGCGAGGAGGA
ATATGACAATGGATCCAAATCATGTGCCAGGGAGTGAACCCAACTTTGCAGCTTA
CAGAACCCAAAGCCGTAGGGGACCGATTGCACGAGAATTTTTCAAGCATTTTGCC
GAAGAGCATGCAGCACATACTGAGAATGCTTGGAGAGATAGGTTTAGGAAGTTT
CTTCTCGCTTACGGCATTGATGATTATATTAGTTATTATGAAGCTGAAAAAGCTCA
GAATAGAGAGCCGGAACCGATGAAGAACCTCACCAATAGACCCAAGAGGCCTGG
CGTTCCCACTCCTGGCAATTATAACTCTGCCGCCAAGAGGGCAAGAAATTATAGT
TCTCAAAGAAATGTTCAGCCTACTGCCAATGCAGCGTCCGCTAATGCTGCTGCCG
CTGCTGCTGCCGCTGCTTCCAACTCTTACGCTATACCAGAAAACGAATTACTGGA

CGAAGATACCATGAATTTCATTTCCAGTTTAAAGAATGATCTATCCAATATTTCAA
ATAGTTTGCCCTTTGAGTATCCACACGAGATTGCGGAAGCTATAAGAAGCGATTT
TTCGAATGAAGATATATGATAATATTGATCCTGATACCATAAGCTTTCCACCA
AAAATTGCAACAACAGATCTTTTCCTGCCATTGTTCTTTCATTTTGGCAGTACGAG
ACAATTTATGGATAAACTTCATGAAGTTATTTCAGGAGATTATGAGCCATCACAG
GCTGAAAAACTGGTACAGGATCTTTGCGATGAAACTGGTATACGTAAAAATTTCA
GTACGAGCATATTGACGTGTTTATCCGGGGATCTGATGGTCTTTCCTCGCTATTTC
TTGAACATGTTTAAGGACAATGTTAATCCTCCTCCCAACGTTCCTGGTATTTGGAC
ACATGATGACGACGAATCGCTAAAAGCAATGATCAAGAACAAATAAGGAAACT
GGTTAAAAAGCATGGAACTGGTAGAATGGAAATGAGGAAAAGATTTTTTGAGAA
GGACCTGTTAATGACTCAATTGAACGGATCTGCTAGCGGAGGTTCTGGCGCGCCA
ACCGGAAATGTGTCCAAGGGTGAAGAATTGTTCACTGGTGTTGTCCCAATTTTGG
TTGAATTAGATGGTGATGTTAATGGGCACAAATTTTCTGTCAGAGGAGAGGGTGA
AGGTGATGCAACAATCGGTAAGTTGACCTTGAAAGTCATTTGCACTACTGGTAAA
CTACCTGTTCCTTGGCCTACCTTGGTCACTACTGTTGGTTATGGTGTTCAATGCTTT
GCTAGATACCCAGATCACATGAAGCAACATGACTTTTACAAGAGTGCCATGCCAG
AAGGTTATGTTCAGGAAAGAACTATCTCCTTCAAAGATGACGGTAAGTACAAGAC
TCGTGCTGAAGTCAAGTTTGAAGGTGATACCTTGGTTAATAGAATCGAGTTAAAG
GGTATTGATTTTAAGGAAGATGGTAACATTTTAGGTCACAAATTGGCTTACAACT
TTAACTCTCACAATGTTTACATCACTGCTGACAAGCAAAAGAACGGTATCAAGGC
TAACTTCAAAATTAGACACAACATTGAGGATGGTGGTGTTCAACTAGCTGACCAT
TATCAACAAAATACTCCAATCGGCGATGGCCCTGTCCTTTTACCAGACAACCATT
ACCTGTCCTACCAATCTCGTGTTTACAAAGATCCAAACGAAAAGAGAGACCACAT
GGCTGTTATTGAGTTTATGACTGCTGCTGGAATTACTCATGGCATGGATGAACTA
CCGCGGAGTGGAGGAACCGGTAGTGGTAACAGATCTTCTAAATAA

REFERENCES

[1] S. H. Askree et al., "A genome-wide screen for Saccharomyces cerevisiae deletion mutants that affect telomere length," Proc. Natl. Acad. Sci. U.S.A., vol. 101, no. 23, pp. 8658-8663, Jun. 2004, doi: 10.1073/pnas.0401263101.

[2] G. H. Romano et al., "Environmental Stresses Disrupt Telomere Length Homeostasis," PLOS Genetics, vol. 9, no. 9, p. e1003721, Sep. 2013, doi: 10.1371/journal.pgen. 1003721.

**Claims**

**1.** A method for determining telomere length comprising the steps of:

(i) providing yeast cells expressing

(ia) a first telomere binding fusion protein comprising a bioluminescence donator, and
(ib) a second telomere binding fusion protein comprising a fluorescence acceptor,

(ii) incubating the yeast cells provided in (i) with a bioluminescence donator substrate,
(iii) detecting bioluminescence of the bioluminescence donator at a wavelength characteristic for the donator and detecting fluorescence of the fluorescence acceptor at a wavelength characteristic for the acceptor (in case of telomeric binding of the first telomere binding fusion protein and the second telomere binding fusion protein in physical proximity), and
(iv) determining the acceptor/donator ratio at both wavelengths, wherein the ratio at said wavelengths is indicative for the telomere length.

2. The method of claim 1, wherein the telomere length is calculated by correlating the ratio of step (iv) to a telomere length using a calibration curve.

3. A method for identifying a substance effecting/having an effect on/influencing telomere length comprising the steps of:

(i) providing yeast cells expressing

(ia) a first telomere binding fusion protein comprising a bioluminescence donator, and
(ib) a second telomere binding fusion protein comprising a fluorescence acceptor,

(ii) incubating the yeast cells provided in (i) with a bioluminescence donator substrate and a substance potentially effecting /having an effect on/influencing telomere length,
(iii) detecting bioluminescence of the bioluminescence donator at a wavelength characteristic for the donator and detecting fluorescence of the fluorescence acceptor at a wavelength characteristic for the acceptor (in case of telomeric binding of the first telomere binding fusion protein and the second telomere binding fusion protein in physical proximity),
(iv) determining the acceptor/donator ratio at both wavelengths, and
(v) comparing the acceptor/donator ratio determined in (iv) with an acceptor/donator reference ratio (obtained under the same conditions but without the substance potentially effecting/having an effect on/influencing telomere length), wherein a difference between the ratio and the reference ratio indicates that the substance effects/has an effect on/influences telomere length.

4. The method of any one of claims 1 to 3, wherein

the first telomere binding fusion protein comprises Rap1-interacting factor 1 (Rifl) or Rap 1-interacting factor 2 (Rif2) and a bioluminescence donator and the second telomere binding fusion protein comprises Rap1 and a fluorescence acceptor, or
the first telomere binding fusion protein comprises Rap1 and a bioluminescence donator and the second telomere binding fusion protein comprises Rif1 or Rif2 and a fluorescence acceptor, wherein preferably
the first telomere binding fusion protein comprises Rif2 and a bioluminescence donator and the second telomere binding fusion protein comprises Rap1 and a fluorescence acceptor.

5. The method of any one of claims 1 to 4, wherein the bioluminescence donator is a luciferase, preferably *Renilla reniforniis-luciferase* (RLuc).

6. The method of any one of claims 1 to 5, wherein the fluorescence acceptor is a yellow fluorescent protein (YFP) or green fluorescent protein 2 (GFP$^2$).

7. The method of any one of claims 1 to 6, wherein the bioluminescence donator substrate is coelenterazine or coelenterazine 400a (DeepBlue C).

8. The method of any one of claims 1 to 7, wherein the yeast cells are *Saccharomyces cerevisiae* cells.

9. The method of any one of claim 3 to 8, wherein

a ratio (acceptor/donator) above the reference ratio (acceptor/donator) indicates that the substance increases telomere length/positively effects telomer length, or

a ratio (acceptor/donator) below the reference ratio (acceptor/donator) indicates that the substance decreases telomere length/negatively effects telomer length.

10. The method of any one of claims 3 to 9, wherein the substance is selected from the group consisting of a drug, foodstuff, food supplement, secondary metabolite of an organism, and another chemical compound, wherein the drug is preferably an anti-cancer drug, a growth factor, G-quadruplex stabiliser/destabiliser, senolytic, senostatic, senomorphic or anti-aging drug/supplement.

11. A yeast cell expressing a first telomere binding fusion protein comprising a bioluminescence donator and a second telomere binding fusion protein comprising a fluorescence acceptor.

12. The yeast cell of claim 11, wherein

the first telomere binding fusion protein comprises Rif1 or Rif2 and a bioluminescence donator and the second telomere binding fusion protein comprises Rap1 and a fluorescence acceptor, or

the first telomere binding fusion protein comprises Rap1 and a bioluminescence donator and the second telomere binding fusion protein comprises Rif1 or Rif2a and a fluorescence acceptor, wherein preferably

the first telomere binding fusion protein comprises Rif2 and a bioluminescence donator and the second telomere binding fusion protein comprises Rap1 and a fluorescence acceptor.

13. The yeast cell of claims 11 or 12, wherein the yeast cell is a *Saccharomyces cerevisiae* cell.

14. Use of the yeast cell of any one of claims 11 to 13 for determining telomere length.

15. Use of the yeast cell of any one of claims 11 to 13 for identifying a substance effecting/having an effect on/influencing telomere length.

FIGURE 1

https://www.berthold.com/en/bioanalytic/knowledge/glossary/bret/

FIGURE 2

Telomeric Repeats

Rif2-RLuc

Rap1-YFP

Emission (535/485 nm)

**FIGURE 3**

**FIGURE 4**

**FIGURE 5**

Europäisches
Patentamt
European
Patent Office
Office européen
des brevets

# EUROPEAN SEARCH REPORT

**Application Number**

EP 24 17 4120

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WOTTON DAVID ET AL: "A novel Rap1p-interacting factor, Rif2p, cooperates with Rif1p to regulate telomere length in Saccharomyces cerevisiae", GENES DEV, vol. 11, no. 6, 15 March 1997 (1997-03-15), pages 748-760, XP093221433, * abstract, pg 751, left col, second par-right col, third par * | 1-15 | INV. C12Q1/25 G01N33/542 |
| Y | US 2003/157519 A1 (ZHANG VIVAN Q [US] ET AL) 21 August 2003 (2003-08-21) * par 0109-0110, 0118-0119, 0293, 0299-0302, 0308-0313; cl 39-41 * | 1-15 | |
| Y | EP 1 088 233 B9 (PERKINELMER BIOSIGNAL INC [CA]; UNIV VANDERBILT [US]) 17 May 2006 (2006-05-17) * par 0053, 0057-0058, 0071, 0156; claims 1-8, 14, 19, 20, 38 * | 1-15 | |
| A | US 2020/215094 A1 (RAMUNAS JOHN [US] ET AL) 9 July 2020 (2020-07-09) * the whole document * | 1-15 | **TECHNICAL FIELDS SEARCHED (IPC)** C12Q G01N |

The present search report has been drawn up for all claims

| Place of search | Date of completion of the search | Examiner |
|---|---|---|
| Munich | 11 November 2024 | Motrescu-Hateley, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P4C01)

## ANNEX TO THE EUROPEAN SEARCH REPORT
## ON EUROPEAN PATENT APPLICATION NO.

EP 24 17 4120

This annex lists the patent family members relating to the patent documents cited in the above-mentioned European search report.
The members are as contained in the European Patent Office EDP file on
The European Patent Office is in no way liable for these particulars which are merely given for the purpose of information.

11-11-2024

| Patent document cited in search report | | | Publication date | Patent family member(s) | | Publication date |
|---|---|---|---|---|---|---|
| US 2003157519 | A1 | | 21-08-2003 | AU | 2002335790 A1 | 28-04-2003 |
| | | | | US | 2003157519 A1 | 21-08-2003 |
| | | | | WO | 03033650 A2 | 24-04-2003 |
| EP 1088233 | B9 | | 17-05-2006 | AU | 4254299 A | 05-01-2000 |
| | | | | CA | 2335305 A1 | 23-12-1999 |
| | | | | DE | 69928255 T2 | 09-11-2006 |
| | | | | EP | 1088233 A2 | 04-04-2001 |
| | | | | WO | 9966324 A2 | 23-12-1999 |
| US 2020215094 | A1 | | 09-07-2020 | AU | 2014218667 A1 | 08-10-2015 |
| | | | | CA | 2902237 A1 | 28-08-2014 |
| | | | | CN | 105164269 A | 16-12-2015 |
| | | | | CN | 117838716 A | 09-04-2024 |
| | | | | CY | 1125092 T1 | 24-03-2023 |
| | | | | DK | 2959005 T3 | 03-01-2022 |
| | | | | EP | 2959005 A1 | 30-12-2015 |
| | | | | EP | 3988112 A1 | 27-04-2022 |
| | | | | ES | 2901402 T3 | 22-03-2022 |
| | | | | FI | 2959005 T3 | 08-10-2024 |
| | | | | HK | 1218934 A1 | 17-03-2017 |
| | | | | HR | P20211842 T1 | 04-03-2022 |
| | | | | HU | E056638 T2 | 28-02-2022 |
| | | | | IL | 296870 A | 01-11-2022 |
| | | | | JP | 7103653 B2 | 20-07-2022 |
| | | | | JP | 7486836 B2 | 20-05-2024 |
| | | | | JP | 2016514953 A | 26-05-2016 |
| | | | | JP | 2019201651 A | 28-11-2019 |
| | | | | JP | 2022141694 A | 29-09-2022 |
| | | | | LT | 2959005 T | 25-01-2022 |
| | | | | PL | 2959005 T3 | 31-01-2022 |
| | | | | PT | 2959005 T | 30-12-2021 |
| | | | | RS | 62824 B1 | 28-02-2022 |
| | | | | RU | 2015140125 A | 28-03-2017 |
| | | | | RU | 2022101438 A | 11-02-2022 |
| | | | | SI | 2959005 T1 | 31-01-2022 |
| | | | | US | 2014242154 A1 | 28-08-2014 |
| | | | | US | 2014242155 A1 | 28-08-2014 |
| | | | | US | 2020215094 A1 | 09-07-2020 |
| | | | | US | 2020215095 A1 | 09-07-2020 |
| | | | | US | 2020281959 A1 | 10-09-2020 |
| | | | | US | 2024277752 A1 | 22-08-2024 |
| | | | | WO | 2014130909 A1 | 28-08-2014 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

*   WO 2024089297 A

*   EP 2023080279 W

**Non-patent literature cited in the description**

*   A multilingual glossary of biotechnological terms: (IUPAC Recommendations). Helvetica Chimica Acta, 1995
*   **S. H. ASKREE et al.** A genome-wide screen for Saccharomyces cerevisiae deletion mutants that affect telomere length. *Proc. Natl. Acad. Sci. U.S.A.*, June 2004, vol. 101 (23), 8658-8663

*   **G. H. ROMANO et al.** Environmental Stresses Disrupt Telomere Length Homeostasis. *PLOS Genetics*, September 2013, vol. 9 (9), e1003721